# EUROPEAN PATENT SPECIFICATION

(11) **EP 4 161 528 B1**
(45) Date of publication and mention of the grant of the patent: **05.11.2025**
(21) Application number: 20781202.5
(22) Date of filing: 14.09.2020
(51) Int. Cl.: A61K 31/538, A61K 31/519, A61P 35/00

(54) **COMBINATION OF RUXOLITINIB WITH INCB057643 FOR USE IN THE TREATMENT OF MYELOPROLIFERATIVE NEOPLASMS**
KOMBINATION VON RUXOLITINIB MIT INCB057643 ZUR VERWENDUNG BEI DER BEHANDLUNG VON MYELOPROLIFERATIVEN NEOPLASMEN
COMBINAISON DE RUXOLITINIB AVEC INCB057643 POUR L'UTILISATION DANS LE TRAITEMENT DE NÉOPLASMES MYÉLOPROLIFÉRATIFS

(30) Priority: 03.06.2020 US 202063034214 P
(43) Date of publication of application: 12.04.2023
(73) Proprietor: Incyte Corporation, Wilmington, DE 19803 (US)
(72) Inventor: STUBBS, Matthew, Wilmington, Delaware 19803 (US); LIU, Phillip C., Wilmington, Delaware 19803 (US)
(74) Representative: Carpmaels & Ransford LLP
(86) International application number: PCT/US2020/050753
(87) International publication number: WO 2021/247064

(56) References cited:
- WO-A1-2015/164480
- FALCHOOK GERALD ET AL: "Development of 2 Bromodomain and Extraterminal Inhibitors With Distinct Pharmacokinetic and Pharmacodynamic Profiles for the Treatment of Advanced Malignancies", CLINICAL CANCER RESEARCH, vol. 26, no. 6, 16 September 2019 (2019-09-16), US, pages 1247 - 1257, XP055773042, ISSN: 1078-0432, Retrieved from the Internet <URL:https://clincancerres.aacrjournals.org/content/clincanres/early/2019/09/14/1078-0432.CCR-18-4071.full.pdf> [retrieved on 202102], DOI: 10.1158/1078-0432.CCR-18-4071
- JIANG QINGFEI ET AL: "BET'ing on Dual JAK/BET Inhibition as a Therapeutic Strategy for Myeloproliferative Neoplasms", CANCER CELL, CELL PRESS, US, vol. 33, no. 1, 8 January 2018 (2018-01-08), pages 3 - 5, XP085334245, ISSN: 1535-6108, DOI: 10.1016/J.CCELL.2017.12.007
- KLEPPE MARIA ET AL: "Dual Targeting of Oncogenic Activation and Inflammatory Signaling Increases Therapeutic Efficacy in Myeloproliferative Neoplasms", CANCER CELL, CELL PRESS, US, vol. 33, no. 1, 14 December 2017 (2017-12-14), pages 29, XP085334235, ISSN: 1535-6108, DOI: 10.1016/J.CCELL.2017.11.009
- D T SAENZ ET AL: "BET protein bromodomain inhibitor-based combinations are highly active against post-myeloproliferative neoplasm secondary AML cells", LEUKEMIA, vol. 31, no. 3, 28 September 2016 (2016-09-28), London, pages 678 - 687, XP055479909, ISSN: 0887-6924, DOI: 10.1038/leu.2016.260
- DATABASE WPI [online] Derwent; 1 January 2019 (2019-01-01), DEMARIO M ET AL: "Use of bromodomain and extra-terminal (BET) inhibitor and a proteasome inhibitor as a medicament for treating multiple myeloma", XP055773658, Database accession no. 2019-882113

## Description

### TECHNICAL FIELD

The present application relates to the JAK1/JAK2 inhibitor, ruxolitinib, in combination with a BET protein inhibitor, 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one for use in treating myeloproliferative neoplasms, wherein the combination is unexpectedly synergistic.

### BACKGROUND

Compound 1 is a small molecule inhibitor of the acyl binding bromodomains found within Bromodomain and Extraterminal Domain (BET) proteins. The four family members (BRD2, 3, 4, T) facilitate transcription by binding acetylated histones and recruiting transcription initiation and elongation complexes, thereby acting as a link between chromatin and transcriptional activation (see Shi, J., and Vakoc, C.R., "The Mechanisms Behind the Therapeutic Activity of BET Bromodomain Inhibition" Molecular Cell 2014; 54, 728-736).

The BRD4 (and occasionally BRD3) gene is a component of a chromosomal translocation which yields an aggressive subtype of squamous carcinomas termed "NUT midline" carcinoma. These midline carcinomas are defined by t(15; 19) translocations which give rise to the BRD4-NUT oncogene. The resultant fusion protein consists of BRD4 at the N-terminus and NUT (nuclear protein in testis) at the C-terminus. Removal of this protein from chromatin, either by RNA silencing or small molecule inhibitors, results in differentiation and growth arrest in these carcinomas (see Filippakopoulos, P., et al., Selective Inhibition of BET Bromodomains. Nature 2010; 468, 1067-1073).

The BET proteins are thought to exert proliferative effects by facilitating transcription of oncogenes such as c-myc among others. BRD4 inhibiting compounds have been shown to prevent BRD4 from associating with chromatin, including on the c-myc promoter, in multiple myeloma models. This dissociation leads to decreased levels of c-myc expression and decreased cell viability (see Delmore, J.E., et al., "BET Bromodomain Inhibition as a Therapeutic Strategy to Target c-MYC" Cell 2011; 146, 904-917). BRD4 has recently been discovered to bind to "super-enhancers" which are vast transcriptional regulatory elements within genes necessary for cell fate and survival. An example is found in multiple myeloma, where the c-myc gene was found to have a super-enhancer region that was bound by BRD4, giving rise to high levels of c-myc transcription (see Loven, J., et al., "Selective Inhibition of Tumor Oncogenes by Disruption of Super-Enhancers" Cell 2013; 153, 320-334).

The MPLW515L mutation is found in a small percentage of patients with Philadelphia-chromosome negative myeloproliferative neoplasms (MPN). This mutation causes constitutive activation of JAK2, leading to enhanced signaling through its downstream targets and subsequent increased levels of cell proliferation. As there is much overlap between the JAK/STAT signaling pathway and the inflammatory pathways regulated transcriptionally by the BET proteins, combination of BET and JAK inhibitors in MPN may form the bases for a new therapeutic treatment protocol.

G. Falchook et al. ("Development of 2 Bromodomain and Extraterminal Inhibitors With Distinct Pharmacokinetic and Pharmacodynamic Profiles for the Treatment of Advanced Malignancies", Clin Cancer Res. 2020 Mar 15;26(6):1247-1257) reports results from two independent first-in-human phase 1/2 dose-escalation and expansion, safety and tolerability studies of two BET inhibitors.

Q. Jiang et al. ("BET'ing on Dual JAK/BET Inhibition as a Therapeutic Strategy for Myeloproliferative Neoplasms", Cancer Cell. 2018 Jan 8;33(1):3-5) relates to a combination strategy designed to inhibit BET bromodomain and JAK/STAT signaling as a method for inhibiting NF-κB and cytokine production in myeloproliferative neoplasms (MPNs).

There is a need for new therapies to improve patient outcome. This application is directed to this need and others.

### SUMMARY

The invention is defined in the appended claims.

The present application provides, *inter alia,* a compound which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, for use in methods of treating a myeloproliferative neoplasm in a patient in need thereof, the methods comprising administering to said patient 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and ruxolitinib, or a pharmaceutically acceptable salt thereof, wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

The present application also provides a compound which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, for use methods of treating a myeloproliferative neoplasm in a patient in need thereof, the method comprising administering to said patient 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and a dose of from about 5 mg/day to about 60 mg/day on a free base basis of ruxolitinib, or a pharmaceutically acceptable salt thereof, wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

The present application also provides the compounds for use according to the invention as defined in the claims at the doses recited herein for use in treating a myeloproliferative neoplasm, wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

The details of one or more embodiments are set forth in the description below. Other features, objects, and advantages will be apparent from the description and from the claims.

### DESCRIPTION OF THE FIGURES

FIG. 1A is a graph depicting efficacy of Compound 1 in the HEL xenograft model, tumor volume (mm³) vs. days post inoculation.
FIG. 1B is a graph depicting tolerability of Compound 1 in the HEL xenograft model, tumor corrected body weight change (%) vs. days post inoculation.
FIG. 2A is a graph depicting efficacy of the combination of Compound 1 and ruxolitinib in the SET-2 model, tumor volume (mm³) vs. days post inoculation.
FIG. 2B is a graph depicting tolerability of the combination of Compound 1 and ruxolitinib in the SET-2 model, tumor corrected body weight change (%) vs. days post inoculation.
FIG. 3A is a graph depicting efficacy of the combination of Compound 1 and ruxolitinib in the SET-2 model, tumor volume (mm³) vs. days post inoculation.
FIG. 3B is a graph depicting tolerability of the combination of Compound 1 and ruxolitinib in the SET-2 model, tumor corrected body weight change (%) vs. days post inoculation.
FIG. 4A is a graph depicting spleen weight and white blood cell counts as measures of efficacy of Compound 1 and ruxolitinib in the MPLW515L mouse model of MPN.
FIG. 4B is a graph depicting spleen weight and white blood cell counts as measures of efficacy of Compound 1 and ruxolitinib in the MPLW515L mouse model of MPN.
FIG. 5 is a table (Table 3) depicting a summary of Compound 1 pharmacokinetic parameters for Compound 1 as monotherapy (part 1 and 2) at steady-state (cycle 1 day 8).
FIG. 6 is a graph depicting inhibition of cMyc protein expression in the pharmacodynamics spiked cell assay versus plasma levels of Compound 1.
FIG. 7 is a graph depicting Compound 1 steady-state plasma concentrations (mean ± se) in participants following once daily dosing of Compound 1 as monotherapy.
FIG. 8 is a graph depicting probability of response (hyperglycemia) versus Compound 1 steady-state AUC following once daily dosing of Compound 1 as monotherapy.

### DETAILED DESCRIPTION

Any references herein to methods of treatment of the human and/or animal body refer to the compounds, pharmaceuticals, pharmaceutical compositions and medicaments disclosed herein for use in a method for treatment of the human and/or animal body by therapy or for diagnosis.

For the terms *"e.g."* and "such as," and grammatical equivalents thereof, the phrase "and without limitation" is understood to follow unless explicitly stated otherwise.

As used herein, the singular forms "a," "an," and "the" include plural referents unless the context clearly dictates otherwise.

As used herein, the term "about" means "approximately" (*e*.*g*., plus or minus approximately 10% of the indicated value).

As used herein, the phrase "solid form" refers to a compound provided herein in either an amorphous state or a crystalline state ("crystalline form" or "crystalline solid" or "crystalline solid form"), whereby a compound provided herein in a crystalline state may optionally include solvent or water within the crystalline lattice, for example, to form a solvated or hydrated crystalline form. The term "hydrated," as used herein, is meant to refer to a crystalline form that includes water molecules in the crystalline lattice. Example "hydrated" crystalline forms include hemihydrates, monohydrates, dihydrates, and the like. Other hydrated forms such as channel hydrates and the like are also included within the meaning of the term.

The present invention relates to the use of a JAK1/JAK2 inhibitor, ruxolitinib, in combination with a BET protein inhibitor which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, for treatment of a myeloproliferative neoplasm, wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia. Ruxolitinib, (3R)-3-cyclopentyl-3-[4-(7H-pyrrolo[2,3-d]pyrimidin-4-yl)-1H-pyrazol-1-yl]propanenitrile, is an inhibitor of JAK1 and JAK2. The IC₅₀ of ruxolitinib was measured by Assay A *infra* at 1 mM ATP and found to be less than 10 nM at JAK1 and JAK2. Ruxolitinib can be made by the procedure described in US 7,598,257 (Example 67), filed December 12, 2006. Ruxolitinib phosphate can be prepared as described in US 2008/0312259.

It has been demonstrated that BET inhibition inhibits inflammatory signaling in myeloproliferative neoplasms (see Kleppe M, Koche R, Zou L, et al., "Dual targeting of oncogenic activation and inflammatory signaling increases therapeutic efficacy in myeloproliferative neoplasms". Cancer Cell 2018; 33:29-43.e7). In the referenced study, the BET inhibitor in combination with JAK inhibition reduced pathologic cytokine production and overall disease burden. Specifically, in an in vivo model of myeloproliferative neoplasm, combination treatment with ruxolitinib and JQ1, a JAK1/2 and a BET inhibitor respectively, resulted in decreased inflammation, reduced disease burden. The treatment also eliminated fibrosis in myelofibrosis mice, a response not previously observed with single-agent JAK inhibitor therapy. Mechanistically it is proposed that the synergistic effects of JAK and BET inhibitors are mediated by blocking 2 master regulators of pathologic inflammatory signaling, JAK/STAT and NF-kB, respectively. Taken together, these data warrant evaluation of BET inhibitors in malignancies characterized by underlying inflammation, such as myeloproliferative neoplasms, including primary myelofibrosis.

The present application provides a compound which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, for use in a method of treating a myeloproliferative neoplasm selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia in a patient in need thereof, comprising administering to said patient ruxolitinib and 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one (Compound 1 below) which is an inhibitor of BET proteins such as BRD2, BRD3, BRD4, and BRD-t. Compound 1 can be prepared as described in US Patent 9,540,368 or US Patent 10,189,832.

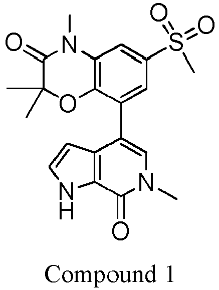

The present application provides a compound which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, for use in a method of treating a myeloproliferative neoplasm in a patient in need thereof, comprising administering to said patient 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and ruxolitinib, or a pharmaceutically acceptable salt thereof, wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia. Administering a combination of Compound 1 and ruxolitinib can provide an enhanced efficacy. As demonstrated by the examples provided herein, combination of Compound 1 and ruxolitinib showed enhanced efficacy in *in vivo* models and significantly (p < 0.05) more efficacious than either ruxolitinib alone or Compound 1 alone. That is, the combination of ruxolitinib and Compound 1 provides efficacy better than the sum of the parts.

The present application further provides a compound which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, for use in a method of treating a myeloproliferative neoplasm in a patient in need thereof, comprising administering to said patient 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and a therapeutically effective amount of ruxolitinib, or a pharmaceutically acceptable salt thereof, wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

The present application further provides compounds for use according to the invention in a method of treating a myeloproliferative neoplasm in a patient in need thereof, comprising administering to said patient a dose from about 2 mg/day to about 20 mg/day on a free base basis of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and a therapeutically effective amount of ruxolitinib, or a pharmaceutically acceptable salt thereof, wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 2 mg/day to about 18 mg/day on a free base basis.

In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 2 mg/day to about 12 mg/day on a free base basis.

In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 4 mg/day to about 8 mg/day on a free base basis.

For example, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 2, about 4, about 6, about 10, about 12, about 14, about 16, about 18, or about 20 mg/day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 2, about 4, about 6, about 8, about 10, about 12, about 14, about 16, about 18, or about 20 mg/day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 2, about 4, about 6, about 8, about 10, or about 12 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 4, about 6, or about 8 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 8, about 10, about 12, about 14, or about 16 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 10, about 12, about 14, about 16 mg, or about 18 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 2 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 4 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 6 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 8 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 10 mg per day on a free base basis. In some embodiments, the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is about 12 mg per day on a free base basis.

In some embodiments, 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and ruxolitinib, or a pharmaceutically acceptable salt thereof are administered once daily (QD). In some embodiments, 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and ruxolitinib, or a pharmaceutically acceptable salt thereof are administered twice daily (BID).

In some embodiments, the dose of ruxolitinib, or a pharmaceutically acceptable salt thereof, is about 5 mg/day to about 60 mg/day on a free base basis. For example, the dose of ruxolitinib, or a pharmaceutically acceptable salt thereof, is about 5, about 10, about 15, about 20, about 25, about 30, about 35, about 40, about 45, about 50, about 55, or 60 mg/day on a free base basis.

In some embodiments, the dose of ruxolitinib, or a pharmaceutically acceptable salt thereof, is about 2.5 mg BID to about 30 mg BID on a free base basis. For example, the dose of ruxolitinib, or a pharmaceutically acceptable salt thereof, is about 2.5, about 5, about 7.5, about 10, about 12.5, about 15, about 17.5, about 20, about 25, or about 30 mg BID on a free base basis.

In some embodiments, the pharmaceutically acceptable salt of ruxolitinib is ruxolitinib phosphate.

In some embodiments, 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is selected from the phosphoric acid salt, dihydrochlorlic acid salt, hydrochloric acid salt, maleic acid salt, adipic acid salt, hydrobromic acid salt, (R)-(-)-mandelic acid salt, salicylic acid salt, benzoic acid salt, benzenesulfonic acid salt, L-pyroglutamic acid salt, methanesulfonic acid salt, (1S)-(+)-10-camphorsulfonic acid salt, fumaric acid salt, sulfuric acid salt, L-tartaric acid salt, and D-tartaric acid salt of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one. In some embodiments, 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one is a free base.

In some embodiments, the 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one includes one or more crystalline solid forms of an inhibitor of a BET protein. A more detailed discussion of Compound 1's crystalline forms can be found in US Patent No. 10,626,114, which is briefly described herein. Typically, different crystalline forms of the same substance have different bulk properties relating to, for example, hygroscopicity, solubility, stability, and the like.

The crystalline solid forms of Compound 1 can include solvent such as water (e.g., a hydrated form) or be substantially free of water and solvent (e.g., forming an anhydrate). In some embodiments, the crystalline solid form is an anhydrate. In further embodiments, the crystalline solid form is hydrated. Compound 1 can be obtained in a solid crystalline form referred to as Form I, Form II, Form III, Form IV, Form V, Form Va, Form VI, Form VII, Form VIII, Form IX, Form X, Form XI, Form XII, Form XIII, Form XIV, and Form X, which are described in US Patent No. 10,626,114.

Experimental data show that Form I is an anhydrate. Form I is characterized by its XRPD pattern and other solid state characteristics. In some embodiments, Form I has one or more characteristic XRPD peaks, in terms of 2-theta, selected from about 8.7, about 9.8, about 12.7, about 21.4, and about 23.3 degrees.

Experimental data show that Form II is an anhydrate. Form II is characterized by its XRPD pattern and other solid state characteristics. In some embodiments, Form II has one or more characteristic XRPD peaks, in terms of 2-theta, selected from about about 6.7, about 9.5, about 10.5, about 14.8, about 16.2, about 17.0, about 18.8, and about 19.3 degrees.

In some embodiments, Form III has one or more characteristic XRPD peaks, in terms of 2-theta, selected from about 7.8, about 12.4, about 13.1, about 15.2, about 15.5, about 16.9, about 17.5, and about 20.3 degrees.

In some embodiments, Form IV has one or more characteristic XRPD peaks, in terms of 2-theta, selected from about 11.2, about 16.3, about 18.7, and about 22.1 degrees.

In some embodiments, Form V has one or more characteristic XRPD peaks, in terms of 2-theta, selected from about 8.2, about 8.5, about 14.1, about 16.3, about 17.1, about 18.9, about 19.8, about 21.8, and about 22.7 degrees.

In some embodiments, Form Va has one or more characteristic XRPD peaks, in terms of 2-theta, selected from about 8.7, about 16.5, about 17.3, about 19.9, and about 21.6 degrees.

In some embodiments, Form VI has one or more characteristic XRPD peaks, in terms of 2-theta, selected from about 8.5, about 9.6, about 11.4, about 12.1, about 13.5, about 14.5, about 15.2, about 17.1, about 17.7, about 18.1, about 19.2, and about 20.7 degrees.

In some embodiments, Form VII has one or more characteristic XRPD peaks, in terms of 2-theta, selected from about 9.9, about 12.2, about 14.8, about 15.7, about 17.0, about 17.5, and about 18.8 degrees.

In some embodiments, Form VIII has one or more characteristic XRPD peaks, in terms of 2-theta, selected from about 8.1, about 8.5, about 16.2, about 16.6, about 17.0, about 17.5, about 18.0, about 18.9, about 19.6, and about 20.1 degrees.

In some embodiments, Form IX has one or more characteristic XRPD peaks, in terms of 2-theta, selected from about 8.6, about 9.1, about 11.4, about 13.4, about 15.2, about 18.2, about 22.1, about 22.8, and about 23.9 degrees.

In some embodiments, Form X has one or more characteristic XRPD peaks, in terms of 2-theta, selected from about 14.9, about 15.3, about 15.8, about 17.0, about 17.7, about 18.3, and about 19.7 degrees.

In some embodiments, Form XI has one or more characteristic XRPD peaks, in terms of 2-theta, selected from about 8.9, about 12.8, about 18.0 about 21.5, about 22.6, and about 23.3 degrees.

In some embodiments, Form XII has one or more characteristic XRPD peaks, in terms of 2-theta, selected from about 5.6, about 11.7, about 13.8, about 14.5, about 16.9, about 17.7, about 18.7, about 23.5, about 24.6, about 34.3, about 44.2, and 44.6 degrees.

In some embodiments, Form XIII has one or more characteristic XRPD peaks, in terms of 2-theta, selected from about 5.7, about 8.6, about 9.8, about 11.8, about 12.6, about 13.4, about 14.1, about 14.8, about 16.6, and about 19.1 degrees.

In some embodiments, Form XIV has one or more characteristic XRPD peaks, in terms of 2-theta, selected from about 4.0, about 11.2, about 11.9, about 14.1, about 14.8, and about 15.9 degrees.

In some embodiments, Form XV has one or more characteristic XRPD peaks, in terms of 2-theta, selected from about 7.4, about 9.6, about 12.4, about 13.4, about 15.5, about 16.9, about 17.7, about 19.0, about 19.5, about 20.6, and about 22.5 degrees.

The present application provides a method of treating a myeloproliferative neoplasm in a patient in need thereof, comprising administering to said patient 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and a therapeutically effective amount of ruxolitinib, or a pharmaceutically acceptable salt thereof, wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

In some embodiments, the myeloproliferative neoplasm is polycythemia vera (PV).

In some embodiments, the myeloproliferative neoplasm is essential thrombocythemia (ET).

In some embodiments, the myeloproliferative neoplasm is primary myelofibrosis.

In some embodiments, the myeloproliferative neoplasm is myelofibrosis.

In some embodiments, ruxolitinib, or pharmaceutically acceptable salt thereof, and 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, are administered orally. In some embodiments, the ruxolitinib, or pharmaceutically acceptable salt thereof, is administered orally. In some embodiments, 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is administered orally.

In some embodiments, ruxolitinib, or pharmaceutically acceptable salt thereof, and 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, are administered simultaneously or sequentially.

The preceding embodiments are intended to be combined in any suitable combination as if the embodiments are multiply dependent claims (e.g., the embodiments related to the individual doses for ruxolitinib, the embodiments related to the individual doses for the BET protein inhibitor (Compound 1), the embodiments related to the salt forms, the embodiments related to the individual types of myeloproliferative neoplasms, and the embodiments related to oral administration can be combined in any combination). The combinations are not separately listed herein merely for the sake of brevity.

All compounds, and pharmaceutically acceptable salts thereof, can be found together with other substances such as water and solvents (*e*.*g*., hydrates and solvates) or can be isolated. When in the solid state, the compounds described herein and salts thereof may occur in various forms and may, *e.g.,* take the form of solvates, including hydrates. The compounds may be in any solid state form, such as a polymorph or solvate, so unless clearly indicated otherwise, reference in the specification to compounds and salts thereof should be understood as encompassing any solid state form of the compound.

The phrase "pharmaceutically acceptable" is employed herein to refer to those compounds, materials, compositions and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The present invention also includes pharmaceutically acceptable salts of the compounds described herein. The term "pharmaceutically acceptable salts" refers to derivatives of the disclosed compounds wherein the parent compound is modified by converting an existing acid or base moiety to its salt form. Examples of pharmaceutically acceptable salts include, but are not limited to, mineral or organic acid salts of basic residues such as amines; alkali or organic salts of acidic residues such as carboxylic acids; and the like. The pharmaceutically acceptable salts of the present invention include the non-toxic salts of the parent compound formed, *e.g.,* from non-toxic inorganic or organic acids. The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, alcohols (*e*.*g*., methanol, ethanol, iso-propanol, or butanol) or acetonitrile (MeCN) are preferred. Lists of suitable salts are found in Remington's Pharmaceutical Sciences, 17th Ed., (Mack Publishing Company, Easton, 1985), p. 1418, Berge et al., J. Pharm. Sci., 1977, 66(1), 1-19, and in Stahl et al., Handbook of Pharmaceutical Salts: Properties, Selection, and Use, (Wiley, 2002). In some embodiments, the compounds described herein include the *N-*oxide forms.

The dosages described herein are on a free base basis. The phrase "on a free base basis" indicates that the amount of the comopund (e.g., ruxolitinib or salt thereof) in the dosage form is measured based on the molecular weight of the comopund free base only, even when the actual active ingredient is a salt of the compound having a different molecular weight than the free base. For example, the conversion factor for ruxolitinib phosphate salt to free base is 0.7575.

The terms "individual" or "patient," used interchangeably, refer to any animal, including mammals, preferably mice, rats, other rodents, rabbits, dogs, cats, swine, cattle, sheep, horses, or primates, and most preferably humans.

The phrase "therapeutically effective amount" refers to the amount of active compound or pharmaceutical agent that elicits the biological or medicinal response in a tissue, system, animal, individual or human that is being sought by a researcher, veterinarian, medical doctor or other clinician.

The term "treating" or "treatment" refers to one or more of (1) inhibiting the disease; *e*.*g*., inhibiting a disease, condition or disorder in an individual who is experiencing or displaying the pathology or symptomatology of the disease, condition or disorder *(i.e.,* arresting further development of the pathology and/or symptomatology); and (2) ameliorating the disease; e.g., ameliorating a disease, condition or disorder in an individual who is experiencing or displaying the pathology or symptomatology of the disease, condition or disorder (*i.e*., reversing the pathology and/or symptomatology) such as decreasing the severity of disease. In one embodiment, treating or treatment includes preventing or reducing the risk of developing the disease; *e.g*., preventing or reducing the risk of developing a disease, condition or disorder in an individual who may be predisposed to the disease, condition or disorder but does not yet experience or display the pathology or symptomatology of the disease.

The term "BID" means two times a day.

The term "QD" means once a day.

### Additional Combinations

Compound 1 can be used in additional combination treatments where Compound 1 is administered in conjunction with other treatments such as the administration of one or more additional therapeutic agents. The additional therapeutic agents are typically those which are normally used to treat the particular condition to be treated. The additional therapeutic agents can include, *e.g*., chemotherapeutics, anti-inflammatory agents, steroids, immunosuppressants, as well as Bcr-Abl, Flt-3, RAF, FAK, and JAK kinase inhibitors for treatment of BET protein-associated diseases, disorders or conditions. The one or more additional pharmaceutical agents can be administered to a patient simultaneously or sequentially.

In some embodiments, Compound 1 can be used in combination with a therapeutic agent that targets an epigenetic regulator. Examples of epigenetic regulators include the histone lysine methyltransferases, histone arginine methyl transferases, histone demethylases, histone deacetylases, histone acetylases, and DNA methyltransferases. Histone deacetylase inhibitors include, *e.g*., vorinostat.

For treating cancer and other proliferative diseases, Compound 1 can be used in combination with chemotherapeutic agents, or other anti-proliferative agents. Compound 1 can also be used in combination with medical therapy such as surgery or radiotherapy, *e.g*., gamma-radiation, neutron beam radiotherapy, electron beam radiotherapy, proton therapy, brachytherapy, and systemic radioactive isotopes. Examples of suitable chemotherapeutic agents include any of: abarelix, aldesleukin, alemtuzumab, alitretinoin, allopurinol, altretamine, anastrozole, arsenic trioxide, asparaginase, azacitidine, bevacizumab, bexarotene, bleomycin, bortezombi, bortezomib, busulfan intravenous, busulfan oral, calusterone, capecitabine, carboplatin, carmustine, cetuximab, chlorambucil, cisplatin, cladribine, clofarabine, cyclophosphamide, cytarabine, dacarbazine, dactinomycin, dalteparin sodium, dasatinib, daunorubicin, decitabine, denileukin, denileukin diftitox, dexrazoxane, docetaxel, doxorubicin, dromostanolone propionate, eculizumab, epirubicin, erlotinib, estramustine, etoposide phosphate, etoposide, exemestane, fentanyl citrate, filgrastim, floxuridine, fludarabine, fluorouracil, fulvestrant, gefitinib, gemcitabine, gemtuzumab ozogamicin, goserelin acetate, histrelin acetate, ibritumomab tiuxetan, idarubicin, ifosfamide, imatinib mesylate, interferon alfa 2a, irinotecan, lapatinib ditosylate, lenalidomide, letrozole, leucovorin, leuprolide acetate, levamisole, lomustine, meclorethamine, megestrol acetate, melphalan, mercaptopurine, methotrexate, methoxsalen, mitomycin C, mitotane, mitoxantrone, nandrolone phenpropionate, nelarabine, nofetumomab, oxaliplatin, paclitaxel, pamidronate, panitumumab, pegaspargase, pegfilgrastim, pemetrexed disodium, pentostatin, pipobroman, plicamycin, procarbazine, quinacrine, rasburicase, rituximab, ruxolitinib, sorafenib, streptozocin, sunitinib, sunitinib maleate, tamoxifen, temozolomide, teniposide, testolactone, thalidomide, thioguanine, thiotepa, topotecan, toremifene, tositumomab, trastuzumab, tretinoin, uracil mustard, valrubicin, vinblastine, vincristine, vinorelbine, vorinostat, and zoledronate.

For treating cancer and other proliferative diseases, Compound 1 can be used in combination with ruxolitinib.

Compound 1 can be used in combination with one or more immune checkpoint inhibitors. Exemplary immune checkpoint inhibitors include inhibitors against immune checkpoint molecules such as CD27, CD28, CD40, CD122, CD96, CD73, CD47, OX40, GITR, CSF1R, JAK, PI3K delta, PI3K gamma, TAM, arginase, CD137 (also known as 4-1BB), ICOS, A2AR, B7-H3, B7-H4, BTLA, CTLA-4, LAG3, TIM3, VISTA, PD-1, PD-L1 and PD-L2. In some embodiments, the immune checkpoint molecule is a stimulatory checkpoint molecule selected from CD27, CD28, CD40, ICOS, OX40, GITR and CD137. In some embodiments, the immune checkpoint molecule is an inhibitory checkpoint molecule selected from A2AR, B7-H3, B7-H4, BTLA, CTLA-4, IDO, KIR, LAG3, PD-1, TIM3, and VISTA. In some embodiments, the compounds provided herein can be used in combination with one or more agents selected from KIR inhibitors, TIGIT inhibitors, LAIR1 inhibitors, CD160 inhibitors, 2B4 inhibitors and TGFR beta inhibitors.

In some embodiments, the inhibitor of an immune checkpoint molecule is anti-PD1 antibody, anti-PD-L1 antibody, or anti-CTLA-4 antibody.

In some embodiments, the inhibitor of an immune checkpoint molecule is an inhibitor of PD-1, e.g., an anti-PD-1 monoclonal antibody. In some embodiments, the anti-PD-1 monoclonal antibody is nivolumab, pembrolizumab (also known as MK-3475), pidilizumab, SHR-1210, PDR001, or AMP-224. In some embodiments, the anti-PD-1 monoclonal antibody is nivolumab or pembrolizumab. In some embodiments, the anti-PD1 antibody is pembrolizumab.

In some embodiments, the inhibitor of an immune checkpoint molecule is an inhibitor of PD-L1, e.g., an anti-PD-L1 monoclonal antibody. In some embodiments, the anti-PD-L1 monoclonal antibody is BMS-935559, MEDI4736, MPDL3280A (also known as RG7446), or MSB0010718C. In some embodiments, the anti-PD-L1 monoclonal antibody is MPDL3280A or MEDI4736.

In some embodiments, the inhibitor of an immune checkpoint molecule is an inhibitor of CTLA-4, e.g., an anti-CTLA-4 antibody. In some embodiments, the anti-CTLA-4 antibody is ipilimumab.

In some embodiments, the inhibitor of an immune checkpoint molecule is an inhibitor of LAG3, e.g., an anti-LAG3 antibody. In some embodiments, the anti-LAG3 antibody is BMS-986016 or LAG525.

In some embodiments, the inhibitor of an immune checkpoint molecule is an inhibitor of GITR, e.g., an anti-GITR antibody. In some embodiments, the anti-GITR antibody is TRX518 or MK-4166.

In some embodiments, the inhibitor of an immune checkpoint molecule is an inhibitor of OX40, e.g., an anti-OX40 antibody or OX40L fusion protein. In some embodiments, the anti-OX40 antibody is MEDI0562. In some embodiments, the OX40L fusion protein is MEDI6383.

Compound 1 can be used in combination with one or more agents for the treatment of diseases such as cancer. In some embodiments, the agent is an alkylating agent, a proteasome inhibitor, a corticosteroid, or an immunomodulatory agent. Examples of an alkylating agent include cyclophosphamide (CY), melphalan (MEL), and bendamustine. In some embodiments, the proteasome inhibitor is carfilzomib. In some embodiments, the corticosteroid is dexamethasone (DEX). In some embodiments, the immunomodulatory agent is lenalidomide (LEN) or pomalidomide (POM).

For treating autoimmune or inflammatory conditions, Compound 1 can be administered in combination with a corticosteroid such as triamcinolone, dexamethasone, fluocinolone, cortisone, prednisolone, or flumetholone.

For treating autoimmune or inflammatory conditions, Compound 1 can be administered in combination with an immune suppressant such as fluocinolone acetonide (Retisert^{®}), rimexolone (AL-2178, Vexol, Alcon), or cyclosporine (Restasis^{®}).

For treating autoimmune or inflammatory conditions, Compound 1 can be administered in combination with one or more additional agents selected from Dehydrex^{™} (Holles Labs), Civamide (Opko), sodium hyaluronate (Vismed, Lantibio/TRB Chemedia), cyclosporine (ST-603, Sirion Therapeutics), ARG101(T) (testosterone, Argentis), AGR1012(P) (Argentis), ecabet sodium (Senju-Ista), gefarnate (Santen), 15-(s)-hydroxyeicosatetraenoic acid (15(S)-HETE), cevilemine, doxycycline (ALTY-0501, Alacrity), minocycline, iDestrin^{™} (NP50301, Nascent Pharmaceuticals), cyclosporine A (Nova22007, Novagali), oxytetracycline (Duramycin, MOLI1901, Lantibio), CF101 (2S, 3S, 4R, 5R)-3, 4-dihydroxy-5-[6-[(3-iodophenyl)methylamino]purin-9-yl]-N-methyl-oxolane-2-carbamyl, Can-Fite Biopharma), voclosporin (LX212 or LX214, Lux Biosciences), ARG103 (Agentis), RX-10045 (synthetic resolvin analog, Resolvyx), DYN15 (Dyanmis Therapeutics), rivoglitazone (DE011, Daiichi Sanko), TB4 (RegeneRx), OPH-01 (Ophtalmis Monaco), PCS101 (Pericor Science), REV1-31 (Evolutec), Lacritin (Senju), rebamipide (Otsuka-Novartis), OT-551 (Othera), PAI-2 (University of Pennsylvania and Temple University), pilocarpine, tacrolimus, pimecrolimus (AMS981, Novartis), loteprednol etabonate, rituximab, diquafosol tetrasodium (INS365, Inspire), KLS-0611 (Kissei Pharmaceuticals), dehydroepiandrosterone, anakinra, efalizumab, mycophenolate sodium, etanercept (Embrel^{®}), hydroxychloroquine, NGX267 (TorreyPines Therapeutics), or thalidomide.

In some embodiments, Compound 1 can be administered in combination with one or more agents selected from an antibiotic, antiviral, antifungal, anesthetic, anti-inflammatory agents including steroidal and non-steroidal anti-inflammatories, and anti-allergic agents. Examples of suitable medicaments include aminoglycosides such as amikacin, gentamycin, tobramycin, streptomycin, netilmycin, and kanamycin; fluoroquinolones such as ciprofloxacin, norfloxacin, ofloxacin, trovafloxacin, lomefloxacin, levofloxacin, and enoxacin; naphthyridine; sulfonamides; polymyxin; chloramphenicol; neomycin; paramomycin; colistimethate; bacitracin; vancomycin; tetracyclines; rifampin and its derivatives ("rifampins"); cycloserine; beta-lactams; cephalosporins; amphotericins; fluconazole; flucytosine; natamycin; miconazole; ketoconazole; corticosteroids; diclofenac; flurbiprofen; ketorolac; suprofen; cromolyn; lodoxamide; levocabastin; naphazoline; antazoline; pheniramine; or azalide antibiotic.

Other examples of agents, one or more of which a provided compound may also be combined with include: a treatment for Alzheimer's Disease such as donepezil and rivastigmine; a treatment for Parkinson's Disease such as L-DOPA/carbidopa, entacapone, ropinirole, pramipexole, bromocriptine, pergolide, trihexyphenidyl, and amantadine; an agent for treating multiple sclerosis (MS) such as beta interferon (*e*.*g*., Avonex^{®} and Rebif^{®}), glatiramer acetate, and mitoxantrone; a treatment for asthma such as albuterol and montelukast; an agent for treating schizophrenia such as zyprexa, risperdal, seroquel, and haloperidol; an anti-inflammatory agent such as a corticosteroid, such as dexamethasone or prednisone, a TNF blocker, IL-1 RA, azathioprine, cyclophosphamide, and sulfasalazine; an immunomodulatory agent, including immunosuppressive agents, such as cyclosporin, tacrolimus, rapamycin, mycophenolate mofetil, an interferon, a corticosteroid, cyclophosphamide, azathioprine, and sulfasalazine; a neurotrophic factor such as an acetylcholinesterase inhibitor, an MAO inhibitor, an interferon, an anti-convulsant, an ion channel blocker, riluzole, or an anti-Parkinson's agent; an agent for treating cardiovascular disease such as a beta-blocker, an ACE inhibitor, a diuretic, a nitrate, a calcium channel blocker, or a statin; an agent for treating liver disease such as a corticosteroid, cholestyramine, an interferon, and an anti-viral agent; an agent for treating blood disorders such as a corticosteroid, an anti-leukemic agent, or a growth factor; or an agent for treating immunodeficiency disorders such as gamma globulin.

In some embodiments, Compound 1 is administered in combination with a JAK kinase inhibitor (e.g., ruxolitinib, tofacitinib, baricitinib, CYT387, GLPG0634, lestaurtinib, pacritinib, TG101348, or a JAK1-selective inhibitor), a Pim kinase inhibitor (including inhibitors of one or more of PIM1, PIM2, and PIM3), a PI3 kinase inhibitor including PI3K-delta selective and broad spectrum PI3K inhibitors, an MEK inhibitor, a cyclin dependent kinase inhibitor, a b-RAF inhibitor, an mTOR inhibitor, a proteasome inhibitor (e.g., bortezomib, carfilzomib), an HDAC-inhibitor (e.g., panobinostat, vorinostat), a DNA methyl transferase inhibitor, dexamethasone, melphalan, or an immunomodulator (e.g., lenolidomide, pomalidomide).

When more than one pharmaceutical agent is administered to a patient, they can be administered simultaneously, sequentially, or in combination (e.g., for more than two agents).

### Compositions

The compounds for use according to the invention can be administered in the form of pharmaceutical compositions. These compositions can be prepared in a manner well known in the pharmaceutical art, and can be administered by a variety of routes, depending upon whether local or systemic treatment is indicated and upon the area to be treated. Administration may be topical (including transdermal, epidermal, ophthalmic and to mucous membranes including intranasal, vaginal and rectal delivery), pulmonary (e.g., by inhalation or insufflation of powders or aerosols, including by nebulizer; intratracheal or intranasal), oral or parenteral. Parenteral administration includes intravenous, intraarterial, subcutaneous, intraperitoneal intramuscular or injection or infusion; or intracranial, e.g., intrathecal or intraventricular, administration. Parenteral administration can be in the form of a single bolus dose, or may be, *e.g.,* by a continuous perfusion pump. Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable.

The pharmaceutical compositions can contain, as the active ingredient, the compounds, or a pharmaceutically acceptable salt thereof, in combination with one or more pharmaceutically acceptable carriers (excipients). In some embodiments, the composition is suitable for topical administration. In making the compositions, the active ingredient is typically mixed with an excipient, diluted by an excipient or enclosed within such a carrier in the form of, *e.g.,* a capsule, sachet, paper, or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing, *e*.*g*., up to 10% by weight of the active compound, soft and hard gelatin capsules, suppositories, sterile injectable solutions and sterile packaged powders.

In preparing a formulation, the active compound can be milled to provide the appropriate particle size prior to combining with the other ingredients. If the active compound is substantially insoluble, it can be milled to a particle size of less than 200 mesh. If the active compound is substantially water soluble, the particle size can be adjusted by milling to provide a substantially uniform distribution in the formulation, *e.g*., about 40 mesh.

The compounds may be milled using known milling procedures such as wet milling to obtain a particle size appropriate for tablet formation and for other formulation types. Finely divided (nanoparticulate) preparations of the compounds of the invention can be prepared by processes known in the art see, *e.g.,* WO 2002/000196.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup and methyl cellulose. The formulations can additionally include: lubricating agents such as talc, magnesium stearate and mineral oil; wetting agents; emulsifying and suspending agents; preserving agents such as methyl- and propylhydroxy-benzoates; and sweetening agents and flavoring agents. The compositions of the invention can be formulated so as to provide quick, sustained or delayed release of the active ingredient after administration to the patient by employing procedures known in the art.

In some embodiments, the pharmaceutical composition comprises silicified microcrystalline cellulose (SMCC) and at least one compound described herein, or a pharmaceutically acceptable salt thereof. In some embodiments, the silicified microcrystalline cellulose comprises about 98% microcrystalline cellulose and about 2% silicon dioxide w/w.

In some embodiments, the composition is a sustained release composition comprising at least one compound described herein, or a pharmaceutically acceptable salt thereof, and at least one pharmaceutically acceptable carrier. In some embodiments, the composition comprises at least one compound described herein, or a pharmaceutically acceptable salt thereof, and at least one component selected from microcrystalline cellulose, lactose monohydrate, hydroxypropyl methylcellulose and polyethylene oxide. In some embodiments, the composition comprises at least one compound described herein, or a pharmaceutically acceptable salt thereof, and microcrystalline cellulose, lactose monohydrate and hydroxypropyl methylcellulose. In some embodiments, the composition comprises at least one compound described herein, or a pharmaceutically acceptable salt thereof, and microcrystalline cellulose, lactose monohydrate and polyethylene oxide. In some embodiments, the composition further comprises magnesium stearate or silicon dioxide. In some embodiments, the microcrystalline cellulose is Avicel PH102^{™}. In some embodiments, the lactose monohydrate is Fast-flo 316^{™}. In some embodiments, the hydroxypropyl methylcellulose is hydroxypropyl methylcellulose 2208 K4M (*e.g*., Methocel K4 M Premier^{™}) and/or hydroxypropyl methylcellulose 2208 K100LV (*e*.*g*., Methocel K00LV^{™}). In some embodiments, the polyethylene oxide is polyethylene oxide WSR 1105 (*e.g.,* Polyox WSR 1105^{™}).

The components used to formulate the pharmaceutical compositions are of high purity and are substantially free of potentially harmful contaminants (e.g., at least National Food grade, generally at least analytical grade, and more typically at least pharmaceutical grade). Particularly for human consumption, the composition is preferably manufactured or formulated under Good Manufacturing Practice standards as defined in the applicable regulations of the U.S. Food and Drug Administration. For example, suitable formulations may be sterile and/or substantially isotonic and/or in full compliance with all Good Manufacturing Practice regulations of the U.S. Food and Drug Administration.

The active compound may be effective over a wide dosage range and is generally administered in a therapeutically effective amount. It will be understood, however, that the amount of the compound actually administered will usually be determined by a physician, according to the relevant circumstances, including the condition to be treated, the chosen route of administration, the actual compound administered, the age, weight and response of the individual patient, the severity of the patient's symptoms and the like.

The therapeutic dosage of a compound of the present invention can vary according to, *e.g.,* the particular use for which the treatment is made, the manner of administration of the compound, the health and condition of the patient, and the judgment of the prescribing physician. The proportion or concentration of a compound of the invention in a pharmaceutical composition can vary depending upon a number of factors including dosage, chemical characteristics (*e.g*., hydrophobicity), and the route of administration.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, the active ingredient is typically dispersed evenly throughout the composition so that the composition can be readily subdivided into equally effective unit dosage forms such as tablets, pills and capsules. This solid preformulation is then subdivided into unit dosage forms of the type described above containing from, e.g., about 0.1 to about 1000 mg of the active ingredient of the present invention.

The tablets or pills of the present invention can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol and cellulose acetate.

The liquid forms in which the compounds and compositions of the present invention can be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described *supra.* In some embodiments, the compositions are administered by the oral or nasal respiratory route for local or systemic effect. Compositions can be nebulized by use of inert gases. Nebulized solutions may be breathed directly from the nebulizing device or the nebulizing device can be attached to a face mask, tent, or intermittent positive pressure breathing machine. Solution, suspension, or powder compositions can be administered orally or nasally from devices which deliver the formulation in an appropriate manner.

Topical formulations can contain one or more conventional carriers. In some embodiments, ointments can contain water and one or more hydrophobic carriers selected from, *e.g.,* liquid paraffin, polyoxyethylene alkyl ether, propylene glycol, white Vaseline^{®} (petroleum jelly) and the like. Carrier compositions of creams can be based on water in combination with glycerol and one or more other components, *e*.*g*., glycerinemonostearate, PEG-glycerinemonostearate and cetylstearyl alcohol. Gels can be formulated using isopropyl alcohol and water, suitably in combination with other components such as, *e.g.,* glycerol, hydroxyethyl cellulose and the like.

The amount of compound or composition administered to a patient will vary depending upon what is being administered, the purpose of the administration, such as prophylaxis or therapy, the state of the patient, the manner of administration and the like. In therapeutic applications, compositions can be administered to a patient already suffering from a disease in an amount sufficient to cure or at least partially arrest the symptoms of the disease and its complications. Effective doses will depend on the disease condition being treated as well as by the judgment of the attending clinician depending upon factors such as the severity of the disease, the age, weight and general condition of the patient and the like.

The compositions administered to a patient can be in the form of pharmaceutical compositions described above. These compositions can be sterilized by conventional sterilization techniques, or may be sterile filtered. Aqueous solutions can be packaged for use as is, or lyophilized, the lyophilized preparation being combined with a sterile aqueous carrier prior to administration. The pH of the compound preparations typically will be between 3 and 11, more preferably from 5 to 9 and most preferably from 7 to 8. It will be understood that use of certain of the foregoing excipients, carriers or stabilizers will result in the formation of pharmaceutical salts.

### Kits

The present application also includes pharmaceutical kits useful, which include one or more containers containing a pharmaceutical composition comprising a therapeutically effective amount of the compound, or any of the embodiments thereof. Such kits can further include one or more of various conventional pharmaceutical kit components, such as, *e.g.,* containers with one or more pharmaceutically acceptable carriers, additional containers, *etc.,* as will be readily apparent to those skilled in the art. Instructions, either as inserts or as labels, indicating quantities of the components to be administered, guidelines for administration, and/or guidelines for mixing the components, can also be included in the kit.

The invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results. The compounds of the Examples have been found to be BET protein-kinase inhibitors according to at least one assay described herein.

### EXAMPLES

### Example 1: Characterization of Compound 1 in Xenograft Models of JAK2V617F-MPN/AML

### Compound and Formulations:

Compound 1 was used in these studies and was manufactured at Incyte Corporation. Ruxolitinib was used in these studies and was manufactured by Wilmington PharmaTech.

### Methods:

Female SCID mice at approximately 5-6 weeks old were subcutaneously inoculated with 107 SET-2 cells in matrigel. Dosing began when tumor size reached approximately 200mm3. Dosing for these studies was performed by oral gavage.

For efficacy studies, measurements of tumors were taken every 2 to 3 days using a digital caliper. Tumor volumes were calculated using the formula: Volume = (length x width2)/2 where width was the smaller dimension. Body weights were also monitored.

Animals were sacrificed (1) at end of study, (2) if tumor size reached 10% of total body weight, or (3) if 20% of body weight is lost.

Statistical analyses were performed using Prism Graphpad software. All comparisons were done using 2-way ANOVA with p< 0.05 deemed significant.

### Efficacy of Compound 1 in the JAK2 V617F Expressing HEL Xenograft Model of Erythroleukemia

The purpose of this study was to examine the ability of the BET inhibitor Compound 1 inhibit tumor growth in an MPN-like AML model expressing the JAK2 V617F mutation. Female SCID mice were subcutaneously inoculated with 10⁷ HEL cells in matrigel. When tumors reached approximately 160 mm³, mice (n = 10 per group) were dosed orally with Compound 1 for 14 days as follows:
1. BID at either 1 or 3 mg/kg, or vehicle control
2. QD at 3, 10, or 30 mg/kg

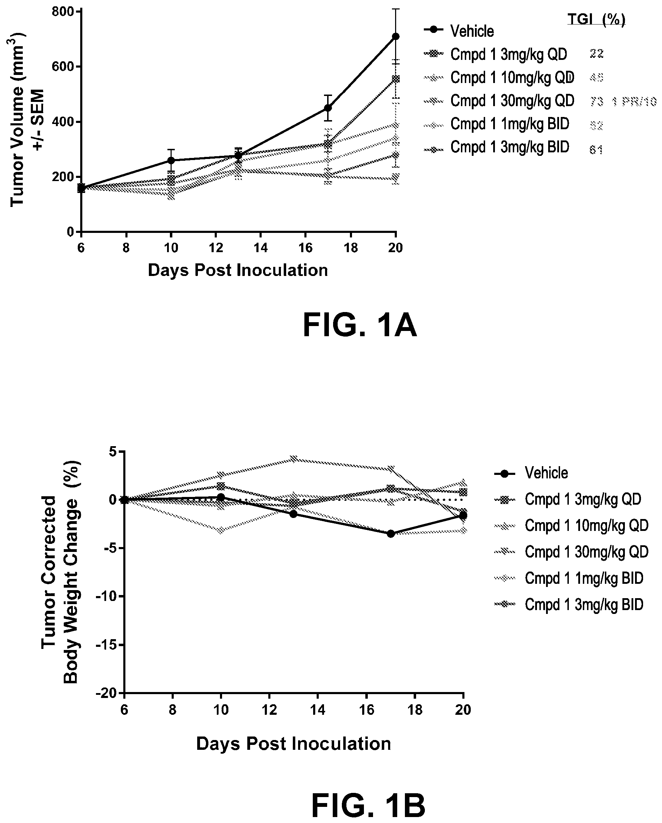

As shown in FIG. 1A, administration of Compound 1 inhibited tumor growth in a dose-related manner, and the mean tumor volumes from each of the Compound 1 treatment groups were statistically significantly reduced from that of the vehicle group (p < 0.02 for each group, 2-way ANOVA). The 30 mg/kg QD dose was the most efficacious and resulted in one statistical tumor regression. The data indicate that dosing Compound 1 on a BID regimen could be considered, but is unnecessary, as QD dosing is as effective as BID dosing in this model. All doses were tolerated as determined by the lack of body weight loss as shown in FIG. 1B.

### Efficacy from combination of Compound 1 and ruxolitinib in the SET-2 xenograft model

Female SCID mice were subcutaneously inoculated with 10⁷ SET-2 cells in matrigel. When tumors reached approximately 175 mm³, mice (n = 8 per group) were dosed orally for 14 days as follows:
1. Vehicle control, or ruxolitinib at 30 mg/kg BID
2. Compound 1 at 10 mg/kg QD
3. Combination of ruxolitinib and Compound 1

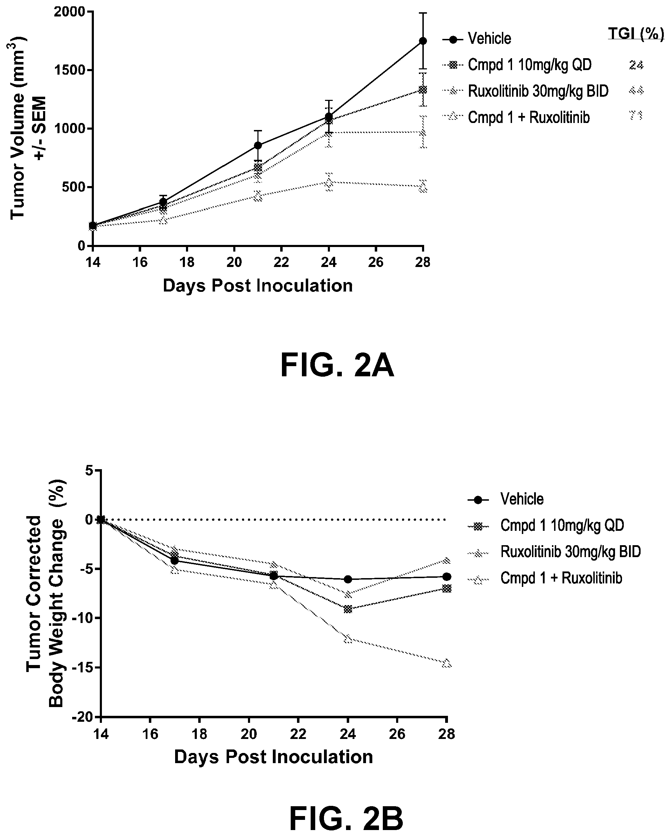

Compound 1 and ruxolitinib were dosed sub-optimally for an optimal read-out of possible combinatorial effects. As seen in FIG. 2A, both monotherapies significantly inhibited tumor growth versus vehicle controls (p < 0.02 for each, 2-way ANOVA). In addition, the combination of Compound 1 and ruxolitinib gave enhanced and significant (p < 0.007 for combination vs. both single agent arms; 2-way ANOVA) efficacy relative to expected outcomes based on single agent efficacies. Approximately 15% body weight loss did occur in the combination group (FIG. 2B), but this nadir was within the limits of tolerability.

### Efficacy from combination of Compound 1 and ruxolitinib in the SET-2 xenograft model

Female SCID mice were subcutaneously inoculated with 10⁷ SET-2 cells in matrigel. When tumors reached approximately 150 mm³, mice (n = 8 per group) were dosed orally for 11 days as follows:
1. Vehicle control, or ruxolitinib at 60 mg/kg BID
2. Compound 1 at 10 mg/kg QD
3. Combination of ruxolitinib and Compound 1

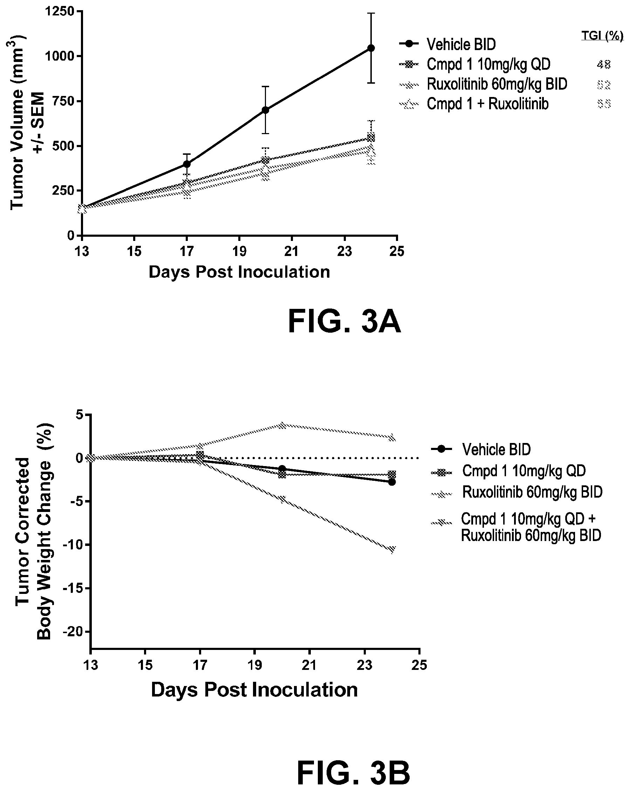

In this study, ruxolitinib was administered at a higher dose than in 1.2, resulting in a significant tumor growth inhibition as a monotherapy (FIG. 3A). Compound 1 administration with ruxolitinib did not increase the tumor growth inhibition seen with single agent ruxolitinib or single agent Compound 1. All doses were tolerated, although the combination of Compound 1 and ruxolitinib did result in body weight loss (FIG. 3B approximately 12%) within the allowable limit.

The BET inhibitor Compound 1 is efficacious at tolerable doses in two models of JAK2V617F AML. Combination of Compound 1 with a sub-optimal dose of the JAK1/2 inhibitor ruxolitinib (30 mg/kg BID) results in a significant increase in tumor growth inhibition over monotherapies. These data demonstrate the potential for BET inhibitors in combination with JAK inhibitors in JAK2V617F driven myeloid neoplasms.

### Example 2: Efficacy of Compound 1 in Combination with Ruxolitinib in a Mouse Model of MPLW515L-Driven MPN

### Compound and Formulations:

Compound 1 and ruxolitinib were used in these studies and were manufactured by Wilmington PharmaTech.

### Methods:

At one week prior to bone marrow transplant, eight week old female Balb/c mice (Charles River Laboratories) were injected once intraperitoneally with 150 mg/kg 5-fluorouracil. Five days later, these mice were sacrificed, and bone marrow was harvested by aspiration of femurs. Red blood cells were lysed using BD Pharma Lyse buffer (BD Biosciences), then washed with PBS. The remaining marrow was plated in 10% FBS/RPMI overnight in standard cell culture incubation. The following day, the bone marrow was infected with ecotropic retrovirus (MSCV puromycin backbone) expressing the MPLW515L gene by adding 1 ml virus to 106 cells, then centrifugation at 1800 rpm, 37°C, 90 minutes in 6 well plates. Following centrifugation, infected cells were kept in the incubator until the following day when they were injected into recipients.

Bone marrow of recipient mice was ablated in eight week old Balb/c mice using 50 mg/kg 5-fluorouracil five days prior to transplantation with Balb/c bone marrow expressing MPLW515L via retroviral infection (approximately 2 x 105 cells per recipient mouse). Blood counts were performed 7 days after transplant and 4 cohorts (n=10 per cohort) with equal mean platelet number were generated for treatment. Dosing commenced on day 8 post implant and continued for 13 days. At day 14 post implant, mice were sacrificed, blood collected by cardiac puncture, and spleens weighed as a surrogate for disease burden.

Blood was collected via by orbital sinus once weekly, and complete blood counts (CBCs) were determined by hematology instrumentation (Abaxis, model HM5).

Statistical analyses were performed using Prism Graphpad software. All comparisons were done using unpaired t tests with p < 0.05 deemed significant.

### Efficacy of Compound 1 in combination with ruxolitinib in the MPLW515L model of mouse MPN

Female Balb/c mice were transplanted with MPLW515L expressing bone marrow in order to assess the ability of the BET inhibitor Compound 1 to improve upon the activity of ruxolitinib alone in a preclinical MPN model. On day 7 post transplant, mice were randomized into cohorts for dosing based on platelet levels, as determined by CBC. The following day oral dosing commenced, with mice receiving Compound 1 at 10 mg/kg QD, ruxolitinib at 60 mg/kg BID, the combination of Compound 1 and ruxolitinib, or vehicle control. Dosing proceeded for 14 days, after which mice were bled for CBC, and spleens were harvested and weighed as a surrogate for disease burden. Two vehicle treated mice died before end of study on day 14 post dosing, one on day 12 of dosing and one on day 13 post dosing.

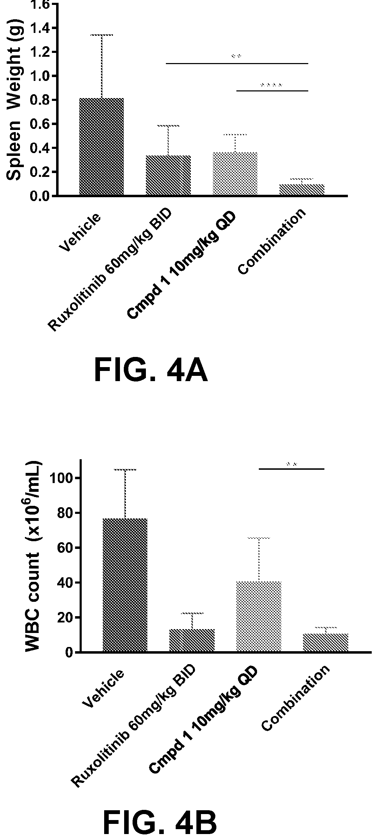

While each single agent was able to reduce spleen size, the combination gave the most efficacy. Mice treated with the Compound 1/ruxolitinib combination had spleens that were significantly smaller than those of either single agent alone (FIG. 4A). Ruxolitinib had a more profound effect on white blood cell count reduction than Compound 1, and there was little difference between WBC counts from ruxolitinib treated mice and mice receiving combination therapy (FIG. 4B).

Statistics were determined by unpaired t tests performed using Prism Graphpad software (** p < 0.01; ****p < 0.0001).

At the doses chosen for this study, both Compound 1 and ruxolitinib were able to slow the expansion of white blood cells and suppress splenomegaly brought on by MPLW515L expressing bone marrow. Combination of Compound 1 with ruxolitinib resulted in a significant decrease in disease burden over either monotherapy as measured by spleen weight. These data demonstrate the potential for BET inhibitors in combination with JAK inhibitors in myeloproliferative neoplasms.

### Example 3: Statistical Demonstration of Synergy Between Compound 1 and Ruxolitinib

The synergy between Compound 1 and ruxolitinib was assessed using the Chou-Talalay equation (see Chou, "Drug Combination Studies and Their Synergy Quantification Using the Chou-Talalay Method" Cancer Res; 70(2) January 15, 2010). Synergy depicts a greater effect with the combination than what would be expected based on how the individual compounds are working. In Table 1, there are data for the two experiments described herein. The first dasa set listed is the MPLW515L model, spleen weights are used as disease progression markers (i.e., bigger spleen = worse disease). MPL SPL depicts the sizes of the spleens in grams from the MPLW515L murine MPN model. Fa shows the fraction affected which is the percentage change in comparison to the vehicle. Expected row shows the expected effect of the combination based on the Chou-Talalay synergy index. Since the number is smaller than the effect in the fraction affected, this demonstrates a larger effect than expected from combining Compound 1 and ruxolitinib demonstrating that synergy is occurring between the two compounds giving an unexpected effect. The second experiment is SET-2 which is the JAK2V617F expressing cell line used in a xenograft tumor model. Fa for SET-2 are the tumor growth inhibition levels for each dose used and the combination. Again the expected value is smaller than the actual effect demonstrating an unexpected synergy. In both experiments, the Chou-Talalay equation gave us a value that was smaller than the effect seen with the combinations of ruxolitinib plus Compound 1, this is interpreted as the combination being synergistic in each experiment.

**Table 1**

| | **Vehicle** | **Ruxolitinib 60mg/kg BID** | **Compound 1 10mg/kg QD** | **Combination** |
|---|---|---|---|---|
| **MPL SPL** | 0.812975 | 0.3367 | 0.3611 | 0.0965 |
| **Fa** | | 0.585842123 | 0.5558289 | 0.881300163 |
| **Expected** | | | | 0.81604304 |
| | | | | |
| **SET-2** | | | | |
| **Fa** | | 0.44 | 0.24 | 0.71 |
| **Expected** | | | | 0.5744 |

### Example 4: Clinical Study Protocol of Compound 1 as Monotherapy in Participants with Myelofibrosis

### Study Design

This is a Phase 1, open-label, 2-part study of Compound 1 as monotherapy in participants with relapsed or refractory myelofibrosis (for further details see "Safety and Tolerability Study of INCB057643 in Participants With Myelofibrosis", ClinicalTrials.gov Identifier: NCT04279847). INCB057643 is also referred to as Compound 1 in the present disclosure. Participants include those who have received at least 1 line of prior therapy including ruxolitinib and have no further available therapy known to provide clinical benefit and with a risk category of intermediate-2 or high according to DIPSS. Participants will receive 4 mg QD of Compound 1 on a continuous basis.

The study will be conducted in 2 parts. Part 1 will evaluate initial safety and tolerability of 4 mg QD of Compound 1 in participants with relapsed or refractory myelofibrosis. Study drug will be self-administered once daily every day for 28 days, which is 1 cycle. Participants will continue taking study drug as long as benefit is derived and discontinuation criteria is not met.

If the starting dose of 4 mg QD is deemed tolerable in Part 1, the starting dose in Part 2 will be 4 mg QD; if not, the starting dose in Part 2 will be 2 mg QD. Part 2 will administer Compound 1 as monotherapy as well.

Throughout the whole study, in both Part 1 and Part 2, AEs will be continuously monitored. If the cumulative incidence of Grade 3 or 4 drug-related AEs occurs in > 40% of participants, the study will be stopped. Likewise, if there is more than 1 fatal drug-related event, the study will be stopped. The study may only be restarted after discussion with the FDA.

### Pharmacokinetics of Compound 1 as Monotherapy

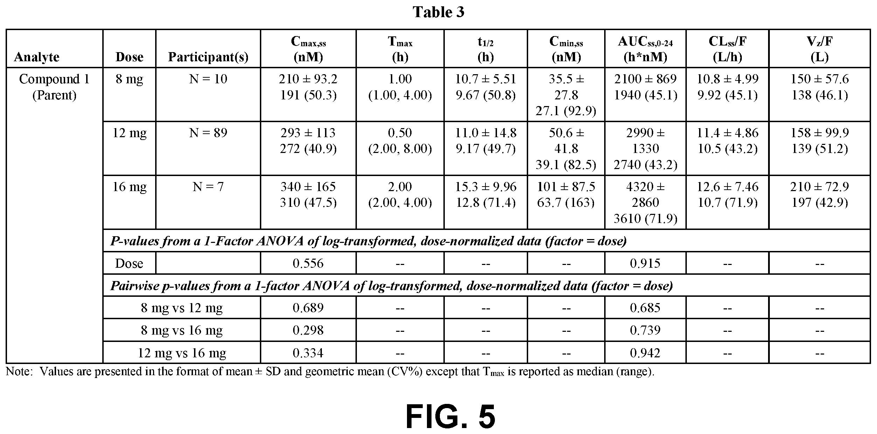

The clinical PK of Compound 1 in participants with advanced malignancies (solid and hematologic malignancies) were evaluated in a Phase 1/2, open-label, dose-escalation/dose-expansion, safety and tolerability study. Participants received continuous QD doses of Compound 1. As of the data cutoff date (23 SEP 2019), 25, 101, and 8 participants who received 8 mg, 12 mg, and 16 mg, respectively, across Part 1 and Part 2 (Compound 1 treated as monotherapy) have been assessed for PK. The PK parameters of parent Compound 1 are summarized in Table 2 and Table 3 (see FIG. 5). Table 2 presents a summary of Compound 1 pharmacokinetic parameters for Compound 1 as monotherapy (part 1 and 2) at cycle 1 day 1. Table 3 presents a summary of Compound 1 pharmacokinetic parameters for Compound 1 as monotherapy (part 1 and 2) at steady-state (cycle 1 day 8).

**Table 2**

| **Analyte** | **Dose** | **Participant(s)** | **Cₘₐₓ (nM)** | **Tₘₐₓ (h)** | **AUC**ₗₐₛₜ **(h*nM)** |
|---|---|---|---|---|---|
| Compound 1 (Parent) | 8 mg | N= 12 | 201 ± 83.6 | 2.00 (1.00, 24.0) | 2310 ± 1120 |
| | | | 188 (38.8) | | 2090 (50.1) |
| | 12 mg | N = 99 | 266 ± 94.7 | 0.50 (2.00, 8.00) | 2740 ± 1090 |
| | | | 249 (39.1) | | 2550 (39.5) |
| | 16 mg | N = 7 | 343 ± 93.2 | 2.00 (2.00, 4.00) | 3370 ± 1060 |
| | | | 329 (34.5) | | 3210 (34.3) |

| | | | | | |
|---|---|---|---|---|---|
| Note: Values are presented in the format of mean ± SD and geometric mean (CV%) except that Tₘₐₓ is reported as median (range). | | | | | |

Blood samples for the determination of plasma concentrations of Compound 1 in Parts 1 and 2 were collected at predose on Cycle 1 Day 1, Cycle 1 Day 2, and Cycle 1 Day 8, and at 0.5, 1, 2, 4, 6, and 8 hours on Cycle 1 Day 1 and Cycle 1 Day 8. The plasma samples of Compound 1 were assayed by a validated LC-MS/MS method.

With multiple-dose administration in the fasted state, Compound 1 plasma concentrations attained the peak values (Cₘₐₓ) typically at 1-2 hours (median Tₘₐₓ) postdose, and subsequently exhibited a monophasic decay, with a steady-state geometric mean t_{½} of approximately 10 hours that was not dose-dependent. The steady-state was predicted to be achieved after 2 days with once daily dosing, based on an effective half-life of 10 hours. There is minimal accumulation of Compound 1 exposure (< 10%) by comparing steady-state AUC₀₋₂₄ to first dose AUC₀₋₂₄. Within the dose range of 8 to 16 mg QD, increases in the Compound 1 steady-state Cₘₐₓ and AUC₀₋₂₄ were proportional to dose, that is, Compound 1 exhibited approximately linear pharmacokinetics over the dose range studied. ANOVA of dose normalized PK parameters (C_{max,ss} and AUC_{ss,0-24}) using overall test or pairwise comparisons (see Table 2) demonstrated that dose normalized C_{max,ss} or AUC_{ss,0-24} was not statistically significantly different (P > 0.05) between or across the doses. Compound 1 exhibited a low steady-state oral clearance with geometric mean CLₛₛ/F of 9.92-10.7 L/h and moderate volume of distribution with geometric mean V_{z}/F of 138-197 L. At a dose of 12 mg QD, the geometric mean (CV%) of t_{½}, steady-state Cₘₐₓ, and AUC₀₋₂₄ were 9.17 h (49.7%), 272 nM (40.9%), and 2740 h*nM (43.2%), respectively.

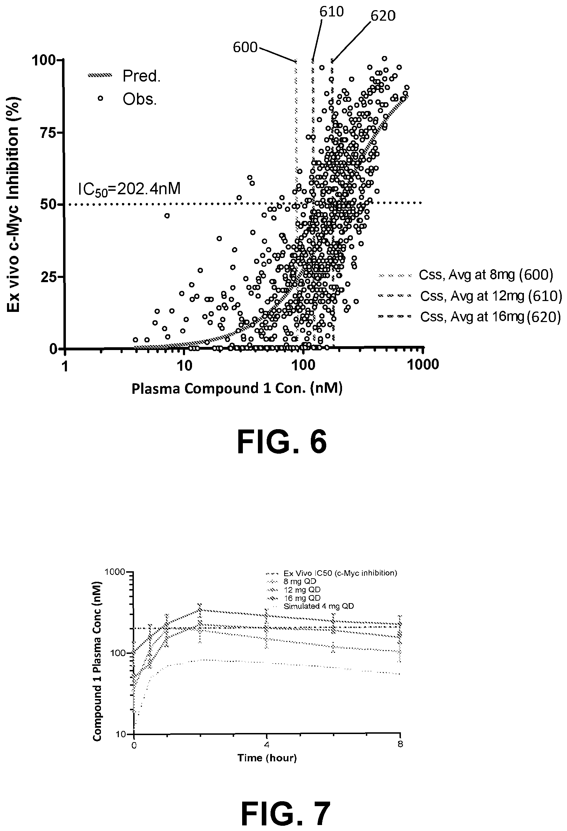

The estimated steady-state AUC₀₋₂₄ of Compound 1 is 457 h*nM and 913 h*nM for 2 mg QD and 4 mg QD, respectively, based on linear PK extrapolation from 12 mg QD geometric mean AUC_{ss,0-24}. The simulated PK data of Compound 1 at 4 mg QD are presented in FIG. 7.

### Pharmacodynamics for the Treatment of Compound 1 as Monotherapy

Pharmacodynamic analysis was performed using an ex vivo assay measuring protein levels of cMyc, a BRD4 target gene, in KMS12BM cells, which were spiked into participant plasma samples collected at predose and various postdose timepoints. In preliminary PD analysis after 8 mg, 12 mg, and 16 mg of oral administration, Compound 1 demonstrated inhibition of total cMyc protein expression with maximal inhibition occurring between 1 and 4 hours. The average cMyc inhibition at steady state (Day 8, from predose to 8 hours) was 30%, 44%, and 65% for the dose of 8 mg (n = 11), 12 mg (n = 51), and 16 mg (n = 7), respectively. Maximum individual peak inhibition of total cMyc protein expression ranged from 16% to 77% at 8 mg, 20% to 92% at 12 mg, and 61% to 97% at 16 mg dose level. Inhibition of cMyc was reduced to < 10% at trough (Cycle 1 Day 8 predose) in the 8-mg and 12-mg cohorts and ~30% in the 16-mg cohort. A composite PK PD curve was plotted for 70 participants, and an IC50 value of 202.4 nM was determined by nonlinear regression curve fitting (see FIG. 6). The ex vivo IC50 coverage over steady-state concentrations of Compound 1 are shown in FIG. 7.

### Relationship of Compound 1 Steady-State AUC and Treatment-Emergent Adverse Events

The selected clinical safety endpoints as deemed appropriate (eg, frequently occurring TEAEs (treatment-emergent adverse events), incident rate > 20% for all AEs (adverse events) and clinically notable adverse events) were used to evaluate exposure-safety relationships. The safety data from a total of 106 participants (including both solid tumors and hematologic malignancies) receiving monotherapy treatment of Compound 1 (n = 10 at 8 mg, n = 89 at 12 mg, and n = 8 at 16 mg) in Parts 1 and 2 were used for analysis. Frequently occurring TEAEs (all grades and causality) in participants who received at least 1 dose of study drug included nausea (47.9%), fatigue (45.5%), decreased appetite (35.5%), thrombocytopenia (28.9%), vomiting (28.1%), anemia (27.3%), diarrhea (27.3%), constipation (20.7%), and dysgeusia (19.8%) as well as clinically notable AEs including hyperglycemia (17.4%), epistaxis (9.9%), INR increased (8.5%), and gastrointestinal hemorrhage (1.9%).

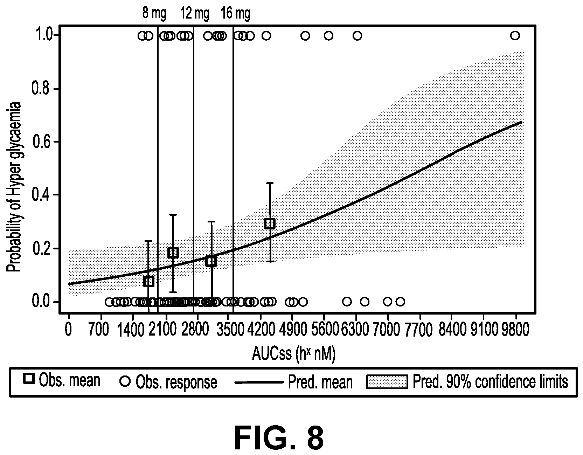

No statistically significant correlation was identified for steady-state Compound 1 AUC and any TEAEs or clinically notable AEs evaluated in this analysis, except hyperglycemia (p-value < 0.05). FIG. 8 shows model-predicted versus observed relationship of Compound 1 steady state AUC and probability of hyperglycemia. The predicted probability of hyperglycemia incidence at dose of 2 mg and 4 mg is 8.0% and 9.2%, respectively, based on AUC and hyperglycemia relationship (Note: Open squares represent first (893-2035 h*nM), second (2074-2605 nM), third (2622-3564 nM), and fourth (3606-9788 nM) quartiles of Compound 1 AUC_{ss,0-24}.). Lack of correlation between Compound 1 AUC_{ss,0-24} and TEAEs could be due to the narrow dose range (8, 12, or 16 mg) explored in this study and small sample sizes in the 8-mg and 16-mg groups of the study (the majority of participants received 12 mg).

Table 4 lists the estimated parameters for Compound 1 AUC and Grade 3 INR (international normalized ratio) increases and Grade 4 gastrointestinal hemorrhage. Two Grade 3 events of INR occurred in participants concomitantly taking warfarin. The steady state AUC of these 2 participants were 6990 h*nM and 9770 h*nM receiving Compound 1 12 mg QD and 16 mg QD, respectively, and the AUCs were much higher than the geometric mean steady-state AUC in 12 mg QD (2740 h*nM) and 16 mg QD (3610 h*nM), respectively.

Two Grade 4 events of gastrointestinal hemorrhage were observed in 2 participants receiving 12 mg QD dose, and the steady-state AUC of these 2 participants were 3470 h*nM and 3610 h*nM, respectively, and the AUCs were slightly higher than geometric mean steady-state AUC in 12 mg QD.

**Table 4**

| **Participants** | **Adverse Event** | **Grade** | **Dose (mg)** | **AUC**_{**ss**,}**₀₋₂₄(h*nM)** |
|---|---|---|---|---|
| 1011 | Gastrointestinal hemorrhage | 4 | 12 | 3470 |
| 9002 | Gastrointestinal hemorrhage | 4 | 12 | 2150 |
| 12005 | INR increased | 3 | 12 | 6990 |
| 16001 | INR increased | 3 | 16 | 9790 |

### Example 5: Efficacy of Compound 1 in Combination with Ruxolitinib in Participants with Myelofibrosis

Preliminary efficacy (spleen length and/or volume reduction) was observed in 2 of 3 myelofibrosis participants treated in the study referenced herein (for further details see "Safety and Tolerability Study of INCB057643 in Participants With Myelofibrosis", ClinicalTrials.gov Identifier: NCT04279847). All three participants were administered ruxolitinib for 6 months prior. A first participant and a second participant had relapsed myelofibrosis which was treated with Compound 1 monotherapy. A third participant was being treated with single-agent ruxolitinib but having suboptimal responses. In reaction to the suboptimal responses, the third participant was then treated with Compound 1 in combination with ruxolitinib.

The first participant received Compound 1 monotherapy 12 mg QD and had a spleen length reduction of 92.5% by spleen palpation (nadir compared to the baseline). The second participant with relapsed myelofibrosis received Compound 1 monotherapy 8 mg QD. The second participant on the lower dose of Compound 1 resulted in only disease progression, and the second participant stayed on study for 24 days. The third participant received Compound 1 8 mg QD in combination with ruxolitinib and experienced a 77% spleen length reduction by spleen palpation and a 44% spleen volume reduction by imaging (nadir compared to the baseline). To put the positive results of the first and third participants in context, when a ruxolitinib monotherapy was employed in clinical trials about 35% reduction in spleen size was observed (for further details see Verstovsek S., Morgan G. "Results of COMFORT- I, a randomized double-blind phase III trial of JAK 1/2 inhibitor INCB18424 (424) vs placebo (PB) for patients with myelofibrosis (MF)", Abstract #6500. 2011 American Society of Clinical Oncology Annual Meeting).

Further preliminary efficacy of a BET inhibitor (CPI-0610) in myelofibrosis participants was also reported in the MANIFEST study (NCT02158858; Hoffman et al 2019, Mascarenhas et al 2019). The enrolled myelofibrosis participants were either refractory, intolerant, or ineligible for ruxolitinib (in the CPI-0610 monotherapy cohort), or were receiving ruxolitinib but experiencing suboptimal response or myelofibrosis progression (in the CPI-0610 and ruxolitinib combination cohort). The participants experienced best spleen volume reduction which ranged from 6%-44% in 10 evaluable participants (monotherapy and combination combined).

## Claims

1. A compound which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, for use in a method of treating a myeloproliferative neoplasm in a patient in need thereof, the method comprising administering to said patient the compound, or a pharmaceutically acceptable salt thereof; and ruxolitinib, or a pharmaceutically acceptable salt thereof;
wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

2. Ruxolitinib, or a pharmaceutically acceptable salt thereof, for use in a method of treating a myeloproliferative neoplasm in a patient in need thereof, the method comprising administering to said patient ruxolitinib, or a pharmaceutically acceptable salt thereof; and a compound which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof;
wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

3. A combination of a compound which is 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, and ruxolitinib, or a pharmaceutically acceptable salt thereof, for use in a method of treating a myeloproliferative neoplasm in a patient in need thereof, the method comprising administering to said patient the compound, or a pharmaceutically acceptable salt thereof, and ruxolitinib, or a pharmaceutically acceptable salt thereof;
wherein the myeloproliferative neoplasm is selected from polycythemia vera (PV), essential thrombocythemia (ET), primary myelofibrosis, chronic myelogenous leukemia (CML), chronic myelomonocytic leukemia (CMML), hypereosinophilic syndrome (HES), systemic mast cell disease (SMCD), chronic neutrophilic leukemia (CNL), and chronic eosinophilic leukemia.

4. The compound, or a pharmaceutically acceptable salt thereof, for use according to claim 1, or ruxolitinib, or a pharmaceutically acceptable salt thereof, for use according to claim 2, or the combination for use according to claim 3, wherein the ruxolitinib, or a pharmaceutically acceptable salt thereof, is ruxolitinib phosphate.

5. The compound, or a pharmaceutically acceptable salt thereof, for use according to claim 1 or 4, or ruxolitinib, or a pharmaceutically acceptable salt thereof, for use according to claim 2 or 4, or the combination for use according to claim 3 or 4, wherein 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one is a solid form.

6. The compound, or a pharmaceutically acceptable salt thereof, for use according to claim 5, or ruxolitinib, or a pharmaceutically acceptable salt thereof, for use according to claim 5, or the combination for use according to claim 5, wherein the solid form is an anhydrate.

7. The compound, or a pharmaceutically acceptable salt thereof, for use according to claim 5, or ruxolitinib, or a pharmaceutically acceptable salt thereof, for use according to claim 5, or the combination for use according to claim 5, wherein the solid form is Form I.

8. The compound, or a pharmaceutically acceptable salt thereof, for use according to claim 7, or ruxolitinib, or a pharmaceutically acceptable salt thereof, for use according to claim 7, or the combination for use according to claim 7, wherein Form I has one or more characteristic XRPD peaks, in terms of 2-theta, selected from about 8.7, about 9.8, about 11.6, about 12.7, about 14.7, about 15.7, about 20.0, about 21.4, about 23.3, and about 27.1 degrees.

9. The compound, or a pharmaceutically acceptable salt thereof, for use according to claim 5, or ruxolitinib, or a pharmaceutically acceptable salt thereof, for use according to claim 5, or the combination for use according to claim 5, the solid form having Form II.

10. The compound, or a pharmaceutically acceptable salt thereof, for use according to claim 9, or ruxolitinib, or a pharmaceutically acceptable salt thereof, for use according to claim 9, or the combination for use according to claim 9, wherein Form II has one or more characteristic XRPD peaks, in terms of 2-theta, selected from about 6.7, about 9.5, about 10.5, about 14.8, about 16.2, about 17.0, about 18.8, and about 19.3 degrees.

11. The compound, or a pharmaceutically acceptable salt thereof, for use according to of any one of claims 1 and 4-10, or ruxolitinib, or a pharmaceutically acceptable salt thereof, for use according to any one of claims 2 and 4-10, or the combination for use according to any one of claims 3-10, wherein the dose of ruxolitinib, or a pharmaceutically acceptable salt thereof, is:
(a) about 5 mg/day to about 60 mg/day; or
(b) about 2.5 mg BID to about 30 mg BID.

12. The compound, or a pharmaceutically acceptable salt thereof, for use according to any one of claims 1 and 4-11, or ruxolitinib, or a pharmaceutically acceptable salt thereof, for use according to any one of claims 2 and 4-11, or the combination for use according to any one of claims 3-11, wherein the dose of 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, is:
(a) about 2 mg/day to about 20 mg/day;
(b) about 2 mg/day to about 18 mg/day;
(c) about 2 mg/day to about 12 mg/day;
(d) about 4 mg/day to about 8 mg/day;
(e) about 2, about 4, about 6, about 8, about 10, about 12, about 14, about 16, about 18, or about 20 mg/day on a free base basis;
(f) about 2 mg/day on a free base basis;
(g) about 4 mg/day on a free base basis;
(h) about 6 mg/day on a free base basis;
(i) about 8 mg/day on a free base basis;
(j) about 10 mg/day on a free base basis; or
(k) about 12 mg/day on a free base basis.

13. The compound, or a pharmaceutically acceptable salt thereof, for use according to any one of claims 1 and 4-12, or ruxolitinib, or a pharmaceutically acceptable salt thereof, for use according to any one of claims 2 and 4-12, or the combination for use according to any one of claims 3-12, wherein:
(a) the myeloproliferative neoplasm is polycythemia vera (PV);
(b) the myeloproliferative neoplasm is essential thrombocythemia (ET); or
(c) the myeloproliferative neoplasm is primary myelofibrosis.

14. The compound, or a pharmaceutically acceptable salt thereof, for use according to any one of claims 1 and 4-13, or ruxolitinib, or a pharmaceutically acceptable salt thereof, for use according to any one of claims 2 and 4-13, or the combination for use according to any one of claims 3-13, wherein ruxolitinib, or pharmaceutically acceptable salt thereof, and 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, are administered orally.

15. The compound, or a pharmaceutically acceptable salt thereof, for use according to any one of claims 1 and 4-14, or ruxolitinib, or a pharmaceutically acceptable salt thereof, for use according to any one of claims 2 and 4-14, or the combination for use according to any one of claims 3-14, wherein ruxolitinib, or pharmaceutically acceptable salt thereof, and 2,2,4-trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-one, or a pharmaceutically acceptable salt thereof, are administered simultaneously or sequentially.

## Patentansprüche

1. Verbindung, bei der es sich um 2,2,4-Trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-on handelt, oder ein pharmazeutisch unbedenkliches Salz davon, zur Verwendung in einem Verfahren zum Behandeln eines myeloproliferativen Neoplasmas bei einem Patienten, der dies benötigt, das Verfahren umfassend das Verabreichen der Verbindung oder eines pharmazeutisch unbedenklichen Salzes davon an den Patienten; und Ruxolitinib oder ein pharmazeutisch unbedenkliches Salz davon;
wobei das myeloproliferative Neoplasma ausgewählt ist aus Polycythaemia vera (PV), essentieller Thrombozythämie (ET), primärer Myelofibrose, chronischer myelogener Leukämie (CML), chronischer myelomonozytischer Leukämie (CMML), hypereosinophilem Syndrom (HES), systemischer Mastzellerkrankung (SMCD), chronischer neutrophiler Leukämie (CNL) und chronischer eosinophiler Leukämie.

2. Ruxolitinib oder ein pharmazeutisch unbedenkliches Salz davon zur Verwendung in einem Verfahren zum Behandeln eines myeloproliferativen Neoplasmas bei einem Patienten, der dies benötigt, das Verfahren umfassend das Verabreichen von Ruxolitinib oder eines pharmazeutisch unbedenklichen Salzes davon an den Patienten; und eine Verbindung, die 2,2,4-Trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-on oder ein pharmazeutisch unbedenkliches Salz davon ist;
wobei das myeloproliferative Neoplasma ausgewählt ist aus Polycythaemia vera (PV), essentieller Thrombozythämie (ET), primärer Myelofibrose, chronischer myelogener Leukämie (CML), chronischer myelomonozytischer Leukämie (CMML), hypereosinophilem Syndrom (HES), systemischer Mastzellerkrankung (SMCD), chronischer neutrophiler Leukämie (CNL) und chronischer eosinophiler Leukämie.

3. Kombination aus einer Verbindung, bei der es sich um 2,2,4-Trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-on oder ein pharmazeutisch unbedenkliches Salz davon und Ruxolitinib oder ein pharmazeutisch unbedenkliches Salz davon handelt, zur Verwendung in einem Verfahren zum Behandeln eines myeloproliferativen Neoplasmas bei einem Patienten, der dies benötigt, das Verfahren umfassend das Verabreichen der Verbindung oder eines pharmazeutisch unbedenklichen Salzes davon und Ruxolitinib oder eines pharmazeutisch unbedenklichen Salzes davon an den Patienten;
wobei das myeloproliferative Neoplasma ausgewählt ist aus Polycythaemia vera (PV), essentieller Thrombozythämie (ET), primärer Myelofibrose, chronischer myelogener Leukämie (CML), chronischer myelomonozytischer Leukämie (CMML), hypereosinophilem Syndrom (HES), systemischer Mastzellerkrankung (SMCD), chronischer neutrophiler Leukämie (CNL) und chronischer eosinophiler Leukämie.

4. Verbindung oder ein pharmazeutisch unbedenkliches Salz davon zur Verwendung nach Anspruch 1 oder Ruxolitinib oder ein pharmazeutisch unbedenkliches Salz davon zur Verwendung nach Anspruch 2 oder die Kombination zur Verwendung nach Anspruch 3, wobei das Ruxolitinib oder ein pharmazeutisch unbedenkliches Salz davon Ruxolitinibphosphat ist.

5. Verbindung oder ein pharmazeutisch unbedenkliches Salz davon zur Verwendung nach Anspruch 1 oder 4 oder Ruxolitinib oder ein pharmazeutisch unbedenkliches Salz davon zur Verwendung nach Anspruch 2 oder 4 oder die Kombination zur Verwendung nach Anspruch 3 oder 4, wobei 2,2,4-Trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-on in einer festen Form vorliegt.

6. Verbindung oder ein pharmazeutisch unbedenkliches Salz davon zur Verwendung nach Anspruch 5 oder Ruxolitinib oder ein pharmazeutisch unbedenkliches Salz davon zur Verwendung nach Anspruch 5 oder die Kombination zur Verwendung nach Anspruch 5, wobei die feste Form ein Anhydrat ist.

7. Verbindung oder ein pharmazeutisch unbedenkliches Salz davon zur Verwendung nach Anspruch 5 oder Ruxolitinib oder ein pharmazeutisch unbedenkliches Salz davon zur Verwendung nach Anspruch 5 oder die Kombination zur Verwendung nach Anspruch 5, wobei die feste Form Form I ist.

8. Verbindung oder ein pharmazeutisch unbedenkliches Salz davon zur Verwendung nach Anspruch 7 oder Ruxolitinib oder ein pharmazeutisch unbedenkliches Salz davon zur Verwendung nach Anspruch 7 oder die Kombination zur Verwendung nach Anspruch 7, wobei Form I einen oder mehrere charakteristische XRPD-Peaks, in Bezug auf 2-Theta aufweist, ausgewählt aus etwa 8,7, etwa 9,8, etwa 11,6, etwa 12,7, etwa 14,7, etwa 15,7, etwa 20,0, etwa 21,4, etwa 23,3 und etwa 27,1 Grad.

9. Verbindung oder ein pharmazeutisch unbedenkliches Salz davon zur Verwendung nach Anspruch 5 oder Ruxolitinib oder ein pharmazeutisch unbedenkliches Salz davon zur Verwendung nach Anspruch 5 oder die Kombination zur Verwendung nach Anspruch 5, wobei die feste Form Form II aufweist.

10. Verbindung oder ein pharmazeutisch unbedenkliches Salz davon zur Verwendung nach Anspruch 9 oder Ruxolitinib oder ein pharmazeutisch unbedenkliches Salz davon zur Verwendung nach Anspruch 9 oder die Kombination zur Verwendung nach Anspruch 9, wobei Form II einen oder mehrere charakteristische XRPD-Peaks, in Bezug auf 2-Theta aufweist, ausgewählt aus etwa 6,7, etwa 9,5, etwa 10,5, etwa 14,8, etwa 16,2, etwa 17,0, etwa 18,8 und etwa 19,3 Grad.

11. Verbindung oder ein pharmazeutisch unbedenkliches Salz davon zur Verwendung nach einem der Ansprüche 1 und 4-10 oder Ruxolitinib oder ein pharmazeutisch unbedenkliches Salz davon zur Verwendung nach einem der Ansprüche 2 und 4-10 oder die Kombination zur Verwendung nach einem der Ansprüche 3-10, wobei die Dosis von Ruxolitinib oder dem pharmazeutisch unbedenklichen Salz davon Folgende ist:
(a) etwa 5 mg/Tag bis etwa 60 mg/Tag; oder
(b) etwa 2,5 mg BID bis etwa 30 mg BID.

12. Verbindung oder ein pharmazeutisch unbedenkliches Salz davon zur Verwendung nach einem der Ansprüche 1 und 4-11 oder Ruxolitinib oder ein pharmazeutisch unbedenkliches Salz davon zur Verwendung nach einem der Ansprüche 2 und 4-11 oder die Kombination zur Verwendung nach einem der Ansprüche 3-11, wobei die Dosis von 2,2,4-Trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-on oder von einem pharmazeutisch unbedenklichen Salz davon Folgende ist:
(a) etwa 2 mg/Tag bis etwa 20 mg/Tag;
(b) etwa 2 mg/Tag bis etwa 18 mg/Tag;
(c) etwa 2 mg/Tag bis etwa 12 mg/Tag;
(d) etwa 4 mg/Tag bis etwa 8 mg/Tag;
(e) etwa 2, etwa 4, etwa 6, etwa 8, etwa 10, etwa 12, etwa 14, etwa 16, etwa 18 oder etwa 20 mg/Tag auf Basis der freien Base;
(f) etwa 2 mg/Tag auf Basis der freien Base;
(g) etwa 4 mg/Tag auf Basis der freien Base;
(h) etwa 6 mg/Tag auf Basis der freien Base;
(i) etwa 8 mg/Tag auf Basis der freien Base;
(j) etwa 10 mg/Tag auf Basis der freien Base; oder
(k) etwa 12 mg/Tag auf Basis der freien Base.

13. Verbindung oder ein pharmazeutisch unbedenkliches Salz davon zur Verwendung nach einem der Ansprüche 1 und 4-12 oder Ruxolitinib oder ein pharmazeutisch unbedenkliches Salz davon zur Verwendung nach einem der Ansprüche 2 und 4-12 oder die Kombination zur Verwendung nach einem der Ansprüche 3-12, wobei:
(a) das myeloproliferative Neoplasma Polycythaemia vera (PV) ist;
(b) das myeloproliferative Neoplasma essentielle Thrombozythämie (ET) ist; oder
(c) das myeloproliferative Neoplasma primäre Myelofibrose ist.

14. Verbindung oder ein pharmazeutisch unbedenkliches Salz davon zur Verwendung nach einem der Ansprüche 1 und 4-13 oder Ruxolitinib oder ein pharmazeutisch unbedenkliches Salz davon zur Verwendung nach einem der Ansprüche 2 und 4-13 oder die Kombination zur Verwendung nach einem der Ansprüche 3-13, wobei Ruxolitinib oder ein pharmazeutisch unbedenkliches Salz davon und 2,2,4-Trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-on oder ein pharmazeutisch unbedenkliches Salz davon oral verabreicht werden.

15. Verbindung oder ein pharmazeutisch unbedenkliches Salz davon zur Verwendung nach einem der Ansprüche 1 und 4-14 oder Ruxolitinib oder ein pharmazeutisch unbedenkliches Salz davon zur Verwendung nach einem der Ansprüche 2 und 4-14 oder die Kombination zur Verwendung nach einem der Ansprüche 3-14, wobei Ruxolitinib oder ein pharmazeutisch unbedenkliches Salz davon und 2,2,4-Trimethyl-8-(6-methyl-7-oxo-6,7-dihydro-1H-pyrrolo[2,3-c]pyridin-4-yl)-6-(methylsulfonyl)-2H-1,4-benzoxazin-3(4H)-on oder ein pharmazeutisch unbedenkliches Salz davon gleichzeitig oder nacheinander verabreicht werden.

## Revendications

1. Composé qui est la 2,2,4-triméthyl-8-(6-méthyl-7-oxo-6,7-dihydro-1H-pyrrolo[2,3-c]pyridin-4-yl)-6-(méthylsulfonyl)-2H-1,4-benzoxazine-3(4H)-one, ou sel pharmaceutiquement acceptable correspondant, destiné à être utilisé dans un procédé de traitement d'un néoplasme myéloprolifératif chez un patient qui en a besoin, le procédé comprenant l'administration audit patient du composé, ou d'un sel pharmaceutiquement acceptable correspondant ; et de ruxolitinib, ou d'un sel pharmaceutiquement acceptable correspondant ;
dans lequel le néoplasme myéloprolifératif est choisi parmi la polyglobulie de Vaquez (PV), la thrombocytémie essentielle (TE), la myélofibrose primitive, la leucémie myéloïde chronique (LMC), la leucémie myélomonocytaire chronique (LMMC), le syndrome hyperéosinophilique (SHE), la maladie systémique des mastocytes (SMCD), la leucémie chronique à neutrophiles (LCN) et la leucémie éosinophilique chronique.

2. Ruxolitinib, ou sel pharmaceutiquement acceptable correspondant, destiné à être utilisé dans un procédé de traitement d'un néoplasme myéloprolifératif chez un patient qui en a besoin, le procédé comprenant l'administration audit patient de ruxolitinib, ou d'un sel pharmaceutiquement acceptable correspondant ; et d'un composé qui est la 2,2,4-triméthyl-8-(6-méthyl-7-oxo-6,7-dihydro-1H-pyrrolo[2,3-c]pyridin-4-yl)-6-(méthylsulfonyl)-2H-1,4-benzoxazine-3(4H)-one, ou d'un sel pharmaceutiquement acceptable correspondant ;
dans lequel le néoplasme myéloprolifératif est choisi parmi la polyglobulie de Vaquez (PV), la thrombocytémie essentielle (TE), la myélofibrose primitive, la leucémie myéloïde chronique (LMC), la leucémie myélomonocytaire chronique (LMMC), le syndrome hyperéosinophilique (SHE), la maladie systémique des mastocytes (SMCD), la leucémie chronique à neutrophiles (LCN) et la leucémie éosinophilique chronique.

3. Combinaison d'un composé qui est la 2,2,4-triméthyl-8-(6-méthyl-7-oxo-6,7-dihydro-1H-pyrrolo[2,3-c]pyridin-4-yl)-6-(méthylsulfonyl)-2H-1,4-benzoxazine-3(4H)-one, ou d'un sel pharmaceutiquement acceptable de celle-ci, et de ruxolitinib, ou d'un sel pharmaceutiquement acceptable correspondant, destinée à être utilisée dans un procédé de traitement d'un néoplasme myéloprolifératif chez un patient qui en a besoin, le procédé comprenant l'administration audit patient du composé, ou d'un sel pharmaceutiquement acceptable correspondant, et de ruxolitinib ou d'un sel pharmaceutiquement acceptable correspondant ;
dans lequel le néoplasme myéloprolifératif est choisi parmi la polyglobulie de Vaquez (PV), la thrombocytémie essentielle (TE), la myélofibrose primitive, la leucémie myéloïde chronique (LMC), la leucémie myélomonocytaire chronique (LMMC), le syndrome hyperéosinophilique (SHE), la maladie systémique des mastocytes (SMCD), la leucémie chronique à neutrophiles (LCN) et la leucémie éosinophilique chronique.

4. Composé, ou sel pharmaceutiquement acceptable correspondant, pour utilisation selon la revendication 1, ou ruxolitinib, ou sel pharmaceutiquement acceptable correspondant, pour utilisation selon la revendication 2, ou combinaison pour utilisation selon la revendication 3, dans lequel/laquelle le ruxolitinib, ou un sel pharmaceutiquement acceptable correspondant, est le phosphate de ruxolitinib.

5. Composé, ou sel pharmaceutiquement acceptable correspondant, pour utilisation selon la revendication 1 ou 4, ou ruxolitinib, ou sel pharmaceutiquement acceptable correspondant, pour utilisation selon la revendication 2 ou 4, ou combinaison pour utilisation selon la revendication 3 ou 4, dans lequel/laquelle la 2,2,4-triméthyl-8-(6-méthyl-7-oxo-6,7-dihydro-1H-pyrrolo[2,3-c]pyridin-4-yl)-6-(méthylsulfonyl)-2H-1,4-benzoxazine-3(4H)-one est une forme solide.

6. Composé, ou sel pharmaceutiquement acceptable correspondant, pour utilisation selon la revendication 5, ou ruxolitinib, ou sel pharmaceutiquement acceptable correspondant, pour utilisation selon la revendication 5, ou combinaison pour utilisation selon la revendication 5, dans lequel/laquelle la forme solide est anhydre.

7. Composé, ou sel pharmaceutiquement acceptable correspondant, pour utilisation selon la revendication 5, ou ruxolitinib, ou sel pharmaceutiquement acceptable correspondant, pour utilisation selon la revendication 5, ou combinaison pour utilisation selon la revendication 5, dans lequel/laquelle la forme solide est la Forme I.

8. Composé, ou sel pharmaceutiquement acceptable correspondant, pour utilisation selon la revendication 7, ou ruxolitinib, ou sel pharmaceutiquement acceptable correspondant, pour utilisation selon la revendication 7, ou combinaison pour utilisation selon la revendication 7, dans lequel/laquelle la Forme I possède un ou plusieurs pics de XRPD caractéristiques, en termes de 2-thêta, choisis parmi environ 8,7, environ 9,8, environ 11,6, environ 12,7, environ 14,7, environ 15,7, environ 20,0, environ 21,4, environ 23,3, et environ 27,1 degrés.

9. Composé, ou sel pharmaceutiquement acceptable correspondant, pour utilisation selon la revendication 5, ou ruxolitinib, ou sel pharmaceutiquement acceptable correspondant, pour utilisation selon la revendication 5, ou combinaison pour utilisation selon la revendication 5, la forme solide ayant la Forme II.

10. Composé, ou sel pharmaceutiquement acceptable correspondant, pour utilisation selon la revendication 9, ou ruxolitinib, ou un sel pharmaceutiquement acceptable correspondant, pour utilisation selon la revendication 9, ou combinaison pour utilisation selon la revendication 9, dans lequel/laquelle la Forme II présente un ou plusieurs pics de XRPD caractéristiques, en termes de 2-thêta, choisis parmi environ 6,7, environ 9,5, environ 10,5, environ 14,8, environ 16,2. environ 17,0, environ 18,8, et environ 19,3 degrés.

11. Composé, ou sel pharmaceutiquement acceptable correspondant, pour utilisation selon l'une quelconque des revendications 1 et 4 à 10, ou ruxolitinib, ou sel pharmaceutiquement acceptable correspondant, pour utilisation selon l'une quelconque des revendications 2 et 4 à 10, ou combinaison pour utilisation selon l'une quelconque des revendications 3 à 10, dans lequel/laquelle la dose de ruxolitinib, ou d'un sel pharmaceutiquement acceptable correspondant, est :
(a) environ 5 mg/jour à environ 60 mg/jour ; ou
(b) environ 2,5 mg 2x/jour à environ 30 mg 2x/jour.

12. Composé, ou sel pharmaceutiquement acceptable correspondant, pour utilisation selon l'une quelconque des revendications 1 et 4 à 11, ou ruxolitinib, ou sel pharmaceutiquement acceptable correspondant, pour utilisation selon l'une quelconque des revendications 2 et 4 à 11, ou combinaison pour utilisation selon l'une quelconque des revendications 3 à 11, dans lequel/laquelle la dose de 2,2,4-triméthyl-8-(6-méthyl-7-oxo-6,7-dihydro-1H-pyrrolo[2,3-c]pyridin-4-yl)-6-(méthylsulfonyl)-2H-1,4-benzoxazine-3(4H)-one, ou d'un sel pharmaceutiquement acceptable correspondant, est :
(a) environ 2 mg/jour à environ 20 mg/jour ;
(b) environ 2 mg/jour à environ 18 mg/jour ;
(c) environ 2 mg/jour à environ 12 mg/jour ;
(d) environ 4 mg/jour à environ 8 mg/jour ;
(e) environ 2, environ 4, environ 6, environ 8, environ 10, environ 12, environ 14, environ 16, environ 18, ou environ 20 mg/jour sur une base de base libre ;
(f) environ 2 mg/jour sur une base de base libre ;
(g) environ 4 mg/jour sur une base de base libre ;
(h) environ 6 mg/jour sur une base de base libre ;
(i) environ 8 mg/jour sur une base de base libre ;
(j) environ 10 mg/jour sur une base de base libre ; ou
(k) environ 12 mg/jour sur une base de base libre.

13. Composé, ou sel pharmaceutiquement acceptable correspondant, pour utilisation selon l'une quelconque des revendications 1 et 4 à 12, ou ruxolitinib, ou sel pharmaceutiquement acceptable correspondant, pour utilisation selon l'une quelconque des revendications 2 et 4 à 12, ou combinaison pour utilisation selon l'une quelconque des revendications 3 à 12, dans lequel/laquelle :
(a) le néoplasme myéloprolifératif est la polyglobulie de Vaquez (PV) ;
(b) le néoplasme myéloprolifératif est une thrombocytémie essentielle (TE) ; ou
(c) le néoplasme myéloprolifératif est une myélofibrose primitive.

14. Composé, ou sel pharmaceutiquement acceptable correspondant, pour utilisation selon l'une quelconque des revendications 1 et 4 à 13, ou ruxolitinib, ou sel pharmaceutiquement acceptable correspondant, pour utilisation selon l'une quelconque des revendications 2 et 4 à 13, ou combinaison pour utilisation selon l'une quelconque des revendications 3 à 13, dans lequel/laquelle le ruxolitinib, ou un sel pharmaceutiquement acceptable correspondant, et la 2,2,4-triméthyl-8-(6-méthyl-7-oxo-6,7-dihydro-1H-pyrrolo[2,3-c]pyridin-4-yl)-6-(méthylsulfonyl)-2H-1,4-benzoxazine-3(4H)-one ou un sel pharmaceutiquement acceptable correspondant, sont administrés par voie orale.

15. Composé, ou sel pharmaceutiquement acceptable correspondant, pour utilisation selon l'une quelconque des revendications 1 et 4 à 14, ou ruxolitinib, ou sel pharmaceutiquement acceptable correspondant, pour utilisation selon l'une quelconque des revendications 2 et 4 à 14, ou combinaison pour utilisation selon l'une quelconque des revendications 3 à 14, dans lequel/laquelle le ruxolitinib, ou un sel pharmaceutiquement acceptable correspondant, et la 2,2,4-triméthyl-8-(6-méthyl-7-oxo-6,7-dihydro-1H-pyrrolo[2,3-c]pyridin-4-yl)-6-(méthylsulfonyl)-2H-1,4-benzoxazine-3(4H)-one ou un sel pharmaceutiquement acceptable de celui-ci, sont administrés simultanément ou séquentiellement.
